# EUROPEAN PATENT APPLICATION

(11) **EP 1 326 076 A2**
(43) Date of publication of application: **09.07.2003**
(21) Application number: 02023774.9
(22) Date of filing: 25.10.2002
(51) Int. Cl.: G01N 33/566

(54) **Methods for indentification of ligands or modulators of 2871 receptor, a g-protein coupled receptor**

(30) Priority: 26.10.2001 US 10546
(71) Applicant: MILLENIUM PHARMACEUTICALS, INC., Cambridge, MA 02139 (US)
(72) Inventor: HODGE, Martin R., Massachussetts, 02420 (US); Jia, Guiquan, Foster City, California 94404 (US)
(74) Representative: Grund, Martin, Dr.

(57) **Abstract**

The present invention relates to ligand identification for the 2871 G-protein-coupled receptor. The invention also relates to methods for identification of agents which can bind 2871 receptor protein and/or modulate 2871 receptor protein function. The invention further relates to agents which bind 2871 receptor protein. The invention further encompasses agents (e.g., agonists and antagonists) which modulate 2871 receptor protein function. The invention further relates to methods of modulating 2871 receptor protein function, and to the use of agents (e.g., agonists and antagonists) in methods for diagnosis and treatment of 2871 receptor related disorders.

## Description

### FIELD OF THE INVENTION

The invention relates to the interaction of 2871 receptor proteins with adenosine nucleotides and/or adenosine nucleotide analogs and to agents (e.g., ligands, antibodies, antagonists, agonists, inhibitors, promoters) such as bioactive lipids, proteolipids or nucleotides, which alter such interactions. The invention provides methods for detecting or identifying an agent that can modulate the binding of 2871 receptor protein to adenosine nucleotides or analogs thereof. Additionally provided are methods for detecting or identifying an agent that can modulate the activity of 2871 receptor protein. The invention further encompasses agonists, and antagonists based on the methods of the invention as well as methods of use in diagnosis and treatment. Further provided are kits comprising reagents useful in conjunction with the methods of the invention.

### BACKGROUND OF THE INVENTION

G-protein coupled receptors (GPCRs) constitute a major class of proteins responsible for transducing a signal within a cell. GPCRs are proteins that mediate signal transduction of a diverse number of ligands through heterotrimeric G proteins *(see, e.g.,* Strader (1994) *Annu. Rev. Biochem.* 63:101-132). GPCRs are a component of many modular cell signaling systems involving, *e.g.,* G proteins, intracellular enzymes and channels. These GPCR-coupled G proteins can modulate the activity of different intracellular effector molecules, *e.g.,* enzymes and ion channels (*see, e.g.,* Gutkind (1998) *J. Biol. Chem.* 273: 1839-1842; Selbie (1998) *Trends Pharmacol. Sci.* 19:87-93).

GPCRs typically include seven transmembrane domains, including an intracellular domain and an extracellular ligand binding domain. The intracellular domain(s) bind G proteins, which represent a family of heterotrimeric proteins comprising of α, β and γ subunits. G proteins typically bind guanine nucleotides. Following ligand binding to the GPCR, a conformational change is transmitted from the extracellular GPCR ligand binding domain to the intracellular domain-bound G protein. This causes the G protein α-subunit to exchange a bound GDP molecule for a GTP molecule and to dissociate from the βγ-subunits. The GTP-bound form of the α-subunit typically functions as an effector-modulating moiety, leading to the production of second messengers, such as, *e*.*g*., cyclic AMP (*e*.*g*., by activation of adenylate cyclase), diacylglycerol or inositol phosphates.

GPCR genes and gene-products are potential causative agents of disease (Spiegel *et al*., *J. Clin. Invest. 92*:1119-1125 (1993); McKusick *et al*., *J. Med*. *Genet. 30*:1-26 (1993)). Specific defects in the rhodopsin gene and the V2 vasopressin receptor gene have been shown to cause various forms of retinitis pigmentosum (Nathans *et al*., *Annu. Rev. Genet. 26*:403-424(1992)) and nephrogenic diabetes insipidus (Holtzman *et al*., *Hum. Mol. Genet. 2*:1201-1204 (1993)), respectively. These receptors are of critical importance to both the central nervous system and peripheral physiological processes. Evolutionary analyses suggest that the ancestor of these proteins originally developed in concert with complex body plans and nervous systems.

A human GPCR termed 2871 receptor has been identified and described in PCT Publication Numbers. WO 99/63087 and WO 01/44474. The 2871 receptor, also identified as GRIR-2, is 358 amino acids in length and has chemical and structural homology to human KIAA0001 and rat VTR 15-20 (U.S. Patent No. 6,063,596). Expression analyses as well as similarity to other receptors has implicated roles of the 2871 receptor in the physiology of various disorders including immune, hematologic, and neoplastic disorders (U.S. Patent No. 6,063,596; and PCT Publication Numbers WO 99/63087; WO 01/44474).

GPCRs, as well as other seven transmembrane proteins, are a major target for drug action and development. Accordingly, it is valuable to the field of pharmaceutical development to identify and characterize GPCRs. Furthermore, it is valuable to identify and characterize ligands for GPCRs in order to aid in the development of drugs for such targets. The present invention advances the state of the art by providing ligands for the previously orphan human GPCR 2871 receptor.

### SUMMARY OF THE INVENTION

The present invention is based, at least in part, on the identification of ligands for the previously orphan human GPCR 2871 receptor protein. Accordingly, the invention is directed to methods of detecting or identifying an agent (e.g., an agonist or antagonist) that binds a 2871 receptor protein or a ligand binding fragment thereof. Such methods typically include a step of combining the agent (e.g., a bioactive lipid, proteolipid or nucleotide) to be tested with a composition comprising 2871 receptor protein or a ligand binding fragment thereof under conditions suitable for binding of a ligand to the 2871 receptor protein. After or simultaneous with the combining step, methods include a step of detecting or measuring the formation of a complex between the agent and the 2871 receptor protein or ligand binding fragment thereof, to thereby identify an agent that binds to the 2871 receptor. In one embodiment, the methods of the invention are competition assays in which the step of combining the agent and the composition containing the 2871 receptor protein or ligand binding fragment thereof is performed in the presence of at least one ligand, for example, a ligand identified as described herein, e.g., an adenosine nucleotide or analog thereof, e.g., AP6A, AMP, AP5A, and AP4A, and ADP-ribose, that binds a 2871 receptor protein or ligand binding fragment thereof.

In the methods of this invention, the compositions comprising the 2871 receptor protein or a ligand binding fragment thereof can comprise, for example, a cell (e.g., primary cell, cell line, recombinant cell) or membrane preparation comprising a 2871 receptor protein or a ligand binding fragment thereof, or a cell or membrane preparation comprising a fusion protein which includes a 2871 receptor protein or ligand binding fragment thereof. To facilitate identification or detection of a complex formed between an agent and a 2871 receptor protein or ligand binding fragment thereof, either the agent, the 2871 receptor protein, or the fusion protein which includes a 2871 receptor protein or ligand binding fragment thereof can include a detectable label (e.g., a radioisotope, an epitope tag, an affinity label, an enzyme, a fluorescent group, a chemiluminescent group, or a chromoluminescent group).

In an additional embodiment, identification or detection of a complex between an agent and a 2871 receptor protein or a fusion protein which includes a 2871 receptor protein or ligand binding fragment thereof can be performed by detecting or measuring a signaling activity or cellular response (e.g., Ca²⁺ flux, pH flux, cAMP flux, chemotaxis, respiratory burst, actin polymerization, shape change, differentiation, proliferation, mediator release or apoptosis) initiated, induced, or promoted by the binding of the agent to the 2871 receptor protein or ligand binding fragment thereof.

Also encompassed by the present invention are methods of detecting or identifying an agent which modulates the binding of an adenosine nucleotide (e.g., AP6A, AMP, AP5A, AP4A, or ADP-ribose) or analog thereof to a 2871 receptor protein or adenosine nucleotide binding fragment thereof. These methods include a step of combining an agent to be tested either simultaneously, before or after addition of an adenosine nucleotide or analog thereof with a composition comprising the 2871 receptor protein or an adenosine nucleotide binding fragment thereof (e.g., a primary cell, cell line, recombinant cell or membrane preparation comprising the protein or fragment thereof), under conditions suitable for binding of the adenosine nucleotide or analog thereof to the receptor protein. Detecting or measuring the formation of a complex between the receptor protein and the adenosine nucleotide or analog thereof, which can be accomplished as described herein, can be performed either concurrently or following the combination step both in the presence and absence of agent to identify a change in the amount of complex formation caused by the agent. A change in the amount of complex formation caused by the addition of agent is thereby indicative that the agent modulates binding of the adenosine nucleotide or analog thereof to the receptor protein. Any of the compounds used in these methods can be labeled as described herein. In one embodiment, the agent to be tested is an antibody or antibody fragment. In another embodiment, the agent to be tested is a small organic molecule.

Additionally provided are methods of identifying a modulator of a 2871 receptor protein. These methods include a step of combining an agent to be tested with a cell expressing a 2871 receptor protein or ligand binding fragment thereof under conditions suitable for detecting a 2871 receptor activity. After, or simultaneous with, the combination step, assessment of the ability of the agent to modulate a 2871 receptor activity can be performed by detecting a change in 2871 receptor activity upon addition of the agent, whereby a change (or modulation) of the 2871 receptor activity by the agent is indicative that the agent is a modulator of the 2871 receptor protein. In one embodiment, the 2871 receptor activity assessed can be a signaling activity or a cellular response such as Ca²⁺ flux, pH flux, cAMP flux, chemotaxis, actin polymerization, shape change, respiratory burst, differentiation, proliferation, mediator release or apoptosis.

The invention further relates to modulators of 2871 receptor protein identified by the methods described herein, which modulate (e.g., inhibit (e.g., an antagonist) or promote(e.g., an agonist)) binding of an adenosine nucleotide or analog thereof to a 2871 receptor protein or ligand-binding fragment thereof. Additionally provided are modulators of 2871 receptor protein identified by the methods described herein, which modulate (e.g., inhibit (e.g., an antagonist) or promote(e.g., an agonist)) function of a 2871 receptor protein or ligand-binding fragment thereof.

In certain embodiments, the modulator can be a small organic molecule. For example, one embodiment relates to a small organic molecule which modulates (e.g., inhibits or promotes) binding of an adenosine nucleotide to a 2871 receptor protein or ligand-binding fragment thereof. A modulator which promotes such binding can be an agonist. A modulator which inhibits such binding can be an antagonist. In certain aspects, the modulator can be an antibody or antigen-binding fragment thereof. In one embodiment, the invention relates to an antibody or antigen-binding fragment thereof which binds a naturally occurring 2871 receptor protein and inhibits binding of a naturally occurring adenosine nucleotide to the receptor.

In one aspect, the modulator relates to an adenosine nucleotide or analog thereof. which modulates the function of 2871 receptor protein or ligand binding fragment thereof upon binding.

The invention also relates to methods for modulating an activity of cells that express a 2871 receptor protein. The methods include contacting cells that express a 2871 receptor protein with an adenosine nucleotide or analog thereof as described herein under conditions sufficient for modulating 2871-induced cellular activity, thereby modulating activity of the cells.

In the methods of the invention, cells that express a 2871 receptor protein can include, for example, recombinant cells or cells which naturally express a 2871 receptor protein such as hematopoeitic cells. Hematopoeitic cells include, red blood cells, macrophages, platelets, polymorphonuclear leucocytes, and lymphocytes. Additional cells which express a 2871 receptor protein include T-helper cells, glial cells, epithelial cells, pancreatic cells, and cardiac cells. Still further, tumorigenic cells which express 2871 receptor protein include breast tumor cells, ovary tumor cells, colon tumor cells, and lung tumor cells. Lung tumor cells can include NCI-H125 lung adenosquamous carcinoma cells. Cellular activities which can be modulated by the present methods include, Ca²⁺ flux, pH flux, cAMP flux, chemotaxis, actin polymerization, shape change, respiratory burst, differentiation, proliferation, mediator release and apoptosis.

Examples of agents which are useful in the modulation of activity of cells that express a 2871 receptor protein include adenosine nucleotides and analogs thereof, e.g., AP6A, AMP, AP5A, AP4A and ADP-ribose.

Additionally, methods for modulating an activity of cells that express a 2871 receptor protein, as described herein, can include contacting cells that express a 2871 receptor protein with an agent that modulates binding of an adenosine nucleotide to 2871 receptor protein under conditions sufficient for modulation, thereby modulating activity of the cells. The methods and the reagents used therein are described herein.

The invention further relates to methods for modulating an activity of cells that express a 2871 receptor protein, as described herein, which include contacting cells that express a 2871 receptor protein with an agent that modulates 2871 receptor protein activity under conditions sufficient for modulation, thereby modulating activity of the cells. The methods and the reagents used therein are described herein.

The invention also relates to therapeutic methods for treating a subject suffering from a 2871 receptor-mediated disorder in which an agent is administered to a subject in need of such therapy. In one embodiment, the method includes administering an effective amount of an agent that modulates binding of an adenosine nucleotide or analog thereof to a 2871 receptor protein. In another embodiment, the method includes administering an effecting amount of an agent that modulates activity of a 2871 receptor protein.

In the therapeutic methods of the invention, the 2871 receptor mediated disorder can include for example, an inflammatory disorder (e.g., chronic obstructive lung disease or asthma, rheumatoid arthritis, multiple sclerosis, or inflammatory bowel disease). In another aspect, the 2871 receptor mediated disorder can include a hematopoietic disorder, such as thrombosis anemia, neutropenia or thrombocytopenia. In another aspect, the 2871 receptor mediated disorder can include a proliferative disorder such as solid tumors (e.g., prostate, colon, lung, breast, ovary, and liver tumors) or hematologic cancers (e.g., leukemia or lymphoma). An agent administered to the subject can include, for example, an adenosine nucleotide or analog thereof; an antibody or antigen-binding fragment thereof which inhibits the binding of an adenosine nucleotide or analog thereof to 2871 receptor protein; or a small organic molecule which has the ability to bind and/or modulate 2871 receptor protein.

Kits comprising reagents for use in conjunction with the methods described herein are also encompassed in the present invention. The reagents incorporated in the kits of the invention can include one or more adenosine nucleotides (e.g., AP6A, AMP, AP5A, AP4A, or ADP-ribose) or analogs thereof that have the ability to bind to a 2871 receptor protein or ligand binding fragment thereof. The reagents can also include means for determining the amount of receptor protein complex in a sample, and means for comparing the amount of receptor protein complex in the sample with a standard. Additionally, the kits of the invention can include instructions for use in the methods of the present invention, and suitable containers for packaging and/or holding reagents.

### DETAILED DESCRIPTION OF THE INVENTION

Described herein are receptor binding and functional studies, the results of which demonstrate that adenosine nucleotides and analogs are novel ligands for the 2871 receptor, also referred to as GRIR-2 (U.S. Patent No. 6,063,596); and that other ligands for 2871 receptor can be agents such as other nucleotides, bioactive lipids, or proteolipids.

The invention relates to the interaction of 2871 receptor protein with adenosine nucleotides and to agents (e.g., ligands, antibodies, antagonists, agonists, inhibitors, promoters) which alter said interaction. Accordingly, the invention relates to methods for detecting or identifying an agent or compound (e.g., a small organic molecule) which can bind to 2871 receptor protein. In another aspect, the invention relates to methods for detecting or identifying an agent or compound which can modulate (i.e., inhibit (reduce or prevent) or promote) the binding of 2871 receptor protein to an adenosine nucleotide. Still another aspect relates to methods for detecting or identifying an agent which can modulate the function of 2871 receptor protein.

As used herein, "2871 receptor polypeptide" or "2871 receptor protein" refers to a naturally occurring 2871 receptor protein (e.g., a protein having the amino acid sequence of the polypeptide in SEQ ID NO:1; see also PCT Publication Numbers WO 99/63087 and WO 01/44474 as well as U.S. Patent No. 6,063,596), a protein having the same amino acid sequence as that of naturally occurring or endogenous 2871 receptor proteins (e.g., recombinant proteins, synthetic proteins), and fragments thereof. Naturally occurring 2871 receptor proteins include wild type proteins such as mature receptor, variants and other isoforms which occur naturally in mammals (e.g., humans, non-human primates, rodents, etc.). Such proteins can be recovered or isolated from a source which naturally produces 2871 receptor protein (e.g., the cells and tissues which express 2871 receptor protein as described herein). These proteins and mammalian receptor proteins having the same amino acid sequence as a naturally occurring or endogenous corresponding mammalian 2871 receptor protein, are referred to by the name of the corresponding mammal. For example, where the corresponding mammal is a human, the protein is designated as a human 2871 receptor protein (e.g., a recombinant human 2871 receptor protein produced in a suitable host cell).

As used herein, a "variant" of 2871 receptor proteins or polypeptides includes a substantially homologous protein encoded by the same genetic locus in an organism, i.e., an allelic variant or polymorphic variant. Variants also encompass proteins derived from other genetic loci in an organism, but having substantial homology to the 2871 receptor protein. Variants also include proteins substantially homologous to the 2871 receptor protein but derived from another organism, i.e., an ortholog. Variants also include proteins that are substantially homologous to the 2871 receptor protein that are produced by chemical synthesis. Variants also include proteins that are substantially homologous to the 2871 receptor protein that are produced by recombinant methods. Variant polypeptides can be fully functional or can lack function in one or more activities. Thus, in the present case, variations can effect the function, for example, of one or more of the regions required for ligand binding.

A variant polypeptide can differ in amino acid sequence by one or more substitutions, deletions, insertions, inversions, fusions, and truncations or a combination of any of these.

Fully functional variants typically contain only conservative variation or variation in non-critical residues or in non-critical regions. Functional variants can also contain substitution of similar amino acids which result in no change or an insignificant change in function. Alternatively, such substitutions may positively or negatively effect function to some degree.

Non-functional variants typically contain one or more non-conservative amino acid substitutions, deletions, insertions, inversions, or truncation or a substitution, insertion, inversion, or deletion in a critical residue or critical region.

As indicated, variants can be naturally-occurring or can be made by recombinant means or chemical synthesis to provide useful and novel characteristics for the receptor polypeptide. This includes preventing immunogenicity from pharmaceutical formulations by preventing protein aggregation.

Useful variants further include those variations resulting in alteration of ligand binding characteristics. For example, one embodiment involves a variation at the binding site that results in binding but not release of ligand. A further useful variation at the same sites can result in a higher affinity for ligand. Useful variants also include changes that provide for affinity for another ligand. Another useful variant includes one that allows binding but which prevents activation by the ligand. Another useful variant includes variation in the transmembrane G-protein-binding/signal transduction domain that provides for reduced or increased binding by the appropriate G-protein or for binding by a different G-protein than the one with which the receptor is normally associated. Another useful variation provides a fusion protein in which one or more domains is operationally fused to one or more domains from another G-protein coupled receptor.

As used herein, two proteins (or a region of the proteins) are substantially homologous when the amino acid sequences are at least about 55, 60, 65%, typically at least about 70-75%, more typically at least about 80-85%, and most typically at least about 90-95% or more homologous. A substantially homologous amino acid sequence, according to the present invention, will be encoded by a nucleic acid sequence hybridizing to the nucleic acid sequence, or portion thereof (e.g., the coding region), of the sequence shown in SEQ ID NO:2 under stringent conditions as more fully described below.

To determine the percent homology of two amino acid sequences, or of two nucleic acids, the sequences are aligned for optimal comparison purposes (e.g., gaps can be introduced in the sequence of one protein or nucleic acid for optimal alignment with the other protein or nucleic acid). The amino acid residues or nucleotides at corresponding amino acid positions or nucleotide positions are then compared. When a position in one sequence is occupied by the same amino acid residue or nucleotide as the corresponding position in the other sequence, then the molecules are homologous at that position. As used herein, amino acid or nucleic acid "homology" is equivalent to amino acid or nucleic acid "identity". The percent homology between the two sequences is a function of the number of identical positions shared by the sequences (i.e., per cent homology equals the number of identical positions/total number of positions times 100). "Similarity" is determined by conserved amino acid substitution. Similar substitutions are those that substitute a given amino acid in a polypeptide by another amino acid of like characteristics. Conservative (e.g., similar) substitutions are likely to be phenotypically silent. Typically seen as conservative substitutions are the replacements, one for another, among the aliphatic amino acids Ala, Val, Leu, and Ile; interchange of the hydroxyl residues Ser and Thr, exchange of the acidic residues Asp and Glu, substitution between the amide residues Asn and Gln, exchange of the basic residues Lys and Arg and replacements among the aromatic residues Phe, Tyr. Guidance concerning which amino acid changes are likely to be phenotypically silent are found in Bowie *et al*., *Science 247*:1306-1310 (1990).

Both identity and similarity can be readily calculated (*Computational Molecular Biology,* Lesk, A.M., ed., Oxford University Press, New York, 1988; *Biocomputing: Informatics and Genome Projects,* Smith, D.W., ed., Academic Press, New York, 1993; *Computer Analysis of Sequence Data*, *Part 1*, Griffin, A.M., and Griffin, H.G., eds., Humana Press, New Jersey, 1994; *Sequence Analysis in Molecular Biology,* von Heinje, G., Academic Press, 1987; and *Sequence Analysis Primer,* Gribskov, M. and Devereux, J., eds., M Stockton Press, New York, 1991).

Preferred computer program methods to determine identity and similarity between two sequences include, but are not limited to, GCG program package (Devereux, J., *et al*., *Nucleic Acids Res. 12(1)*:387 (1984)), BLASTP, BLASTN, FASTA (Atschul, S.F. *et al*., *J. Molec. Biol. 215*:403 (1990)).

Amino acids that are essential for function can be identified by methods known in the art, such as site-directed mutagenesis or alanine-scanning mutagenesis (Cunningham *et al*., *Science 244*:1081-1085 (1989)). The latter procedure introduces single alanine mutations at every residue in the molecule. The resulting mutant molecules are then tested for biological activity such as receptor binding or *in vitro,* or *in vitro* proliferative activity. Sites that are critical for ligand-receptor binding can also be determined by structural analysis such as crystallization, nuclear magnetic resonance or photoaffinity labeling (Smith *et al*., *J. Mol. Biol. 224*:899-904 (1992); de Vos *et al. Science 255*:306-312 (1992)). As indicated herein, highly conserved positive charged residues in the following transmembrane domain-corresponding residue residues may be particularly relevant for targeting for drug development: TM2:8; TM2:24; TM3:1; TM4:4, TM6:23, and TM7:3. See Table 1 below.

Table 1 below depicts an alignment of 2871 receptor protein with additional proteins known in the art having the closest homology. The alignment shows the amino acid sequence alignment for the human 2871 receptor protein (h2871; SEQ ID NO:1) with the murine 2871 receptor protein (m2871; GenBank Accession Number AAK01866; SEQ ID NO:3), murine UDP-glucose receptor (mUDPGR; GenBank Accession Number AAG09275; SEQ ID NO:4), rat UDP-glucose receptor (rUDPGR; GenBank Accession Number AAB71745; SEQ ID NO:5), human UDP-glucose receptor (hUDPGR; GenBank Accession Number KIAA0001; SEQ ID NO:6), the murine ADP receptor 2Y12 (mP2Y12R; GenBank Accession Number AK014807; SEQ ID NO:7); the rat ADP receptor 2Y12 (rP2Y12R; GenBank Accession Number AAG48945; SEQ ID NO:8) human ADP receptor 2Y12 (hP2Y12R; GenBank Accession Number AAG48944; SEQ ID NO:9) the putative murine AP6A receptor (mAP6A; GenBank Accession Number AK008013; SEQ ID NO:10); and the putative human AP6A receptor (GPR86; GenBank Accession Number AAL01038; SEQ ID NO:11). Transmembrane TM domains are in bold italic, conserved K/R are marked (*).

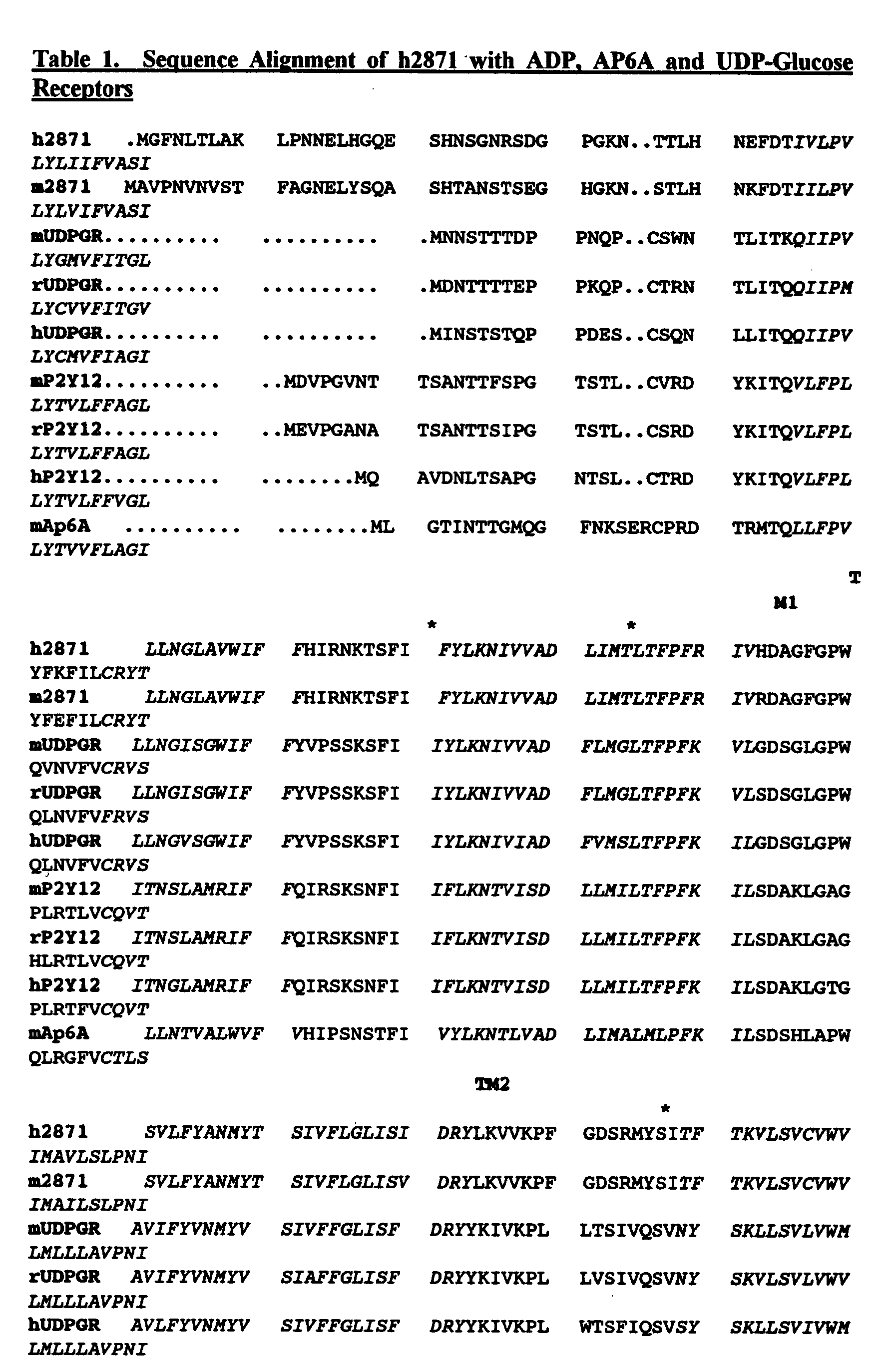

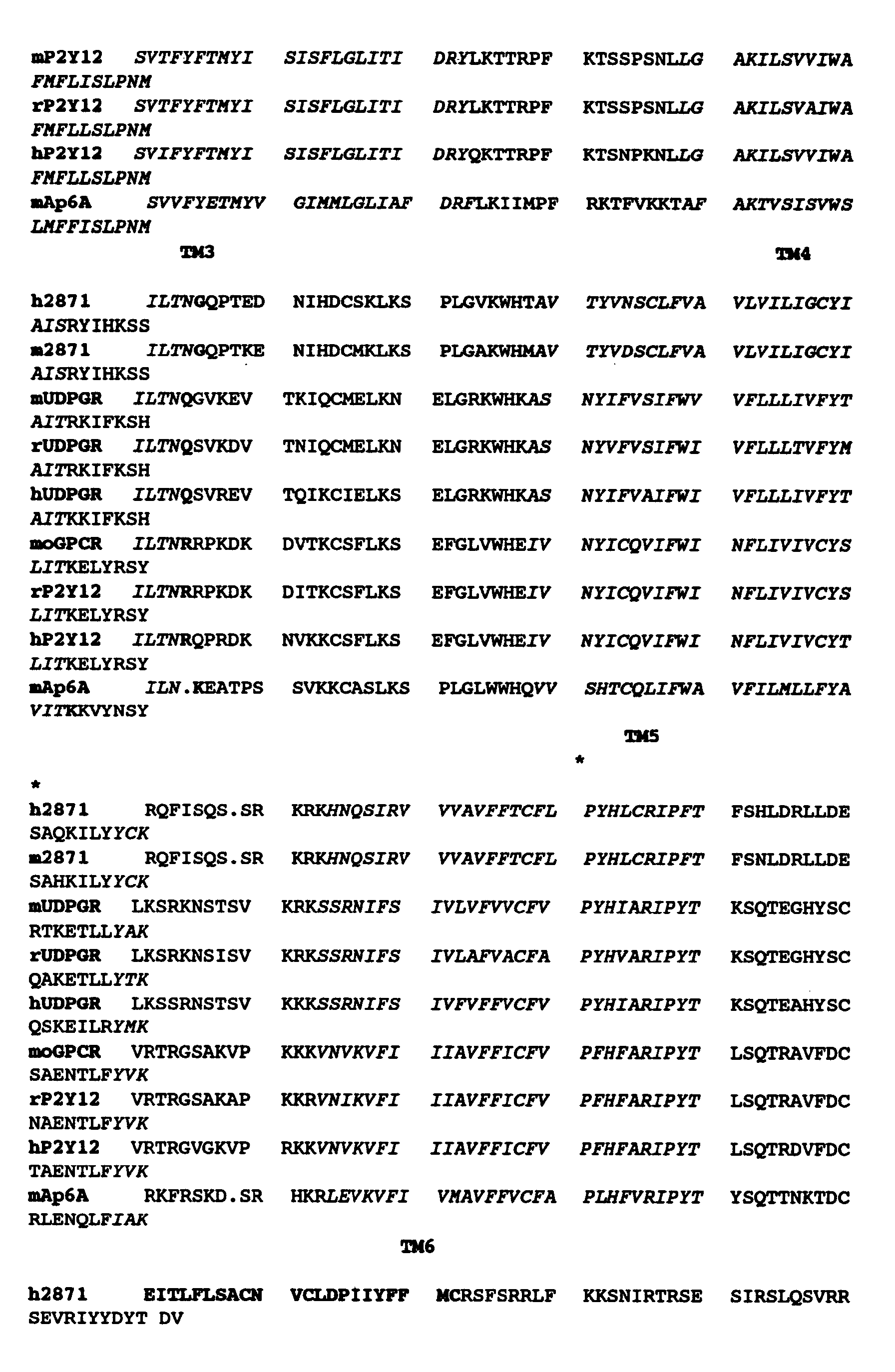

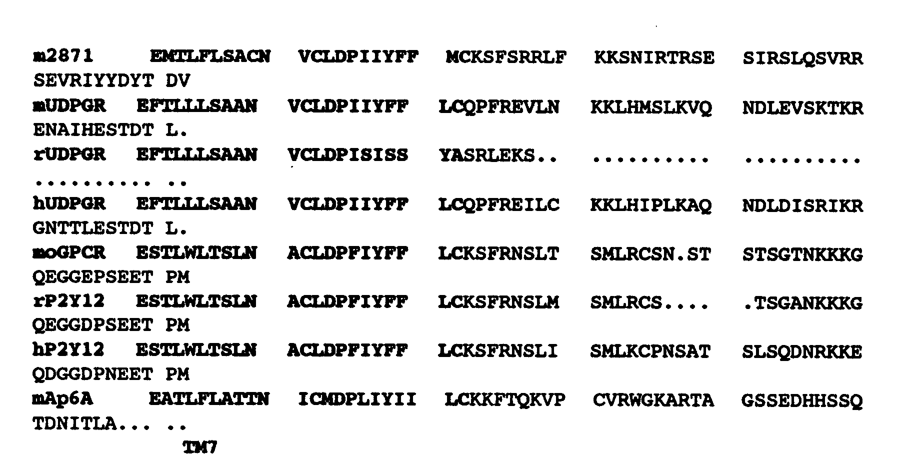

2871 receptor protein has the closest homology to UDP-Glucose receptor (50% identical and 69% similar in N-TM5; 41% identical and 64% similar in TM6-C) and ADP receptor P2Y12, (46% identical, 68% similar). These receptors appear to form a new group that is distinct from other known nucleotide receptors (P2Y1-11). Highly conserved positive charged residues align in the following transmembrane domain-corresponding residue: TM2:8; TM2:24; TM3:1; TM4:4, TM6:23, and TM7:3. These residues may be particularly relevant for targeting for drug development.

The 2871 receptor protein has been identified and described in PCT Publication Numbers. WO 99/63087 and WO 01/44474, as well as U.S. Patent No. 6,063,596, which describes 2871 receptor protein as GRIR-2, the contents of which are incorporated herein by reference.

The 2871 receptor protein has been found to specifically bind adenosine nucleotides. As used herein, adenosine nucleotides refers to nucleotides comprising an adenine base, and can include more than one adenine base (e.g., ATP, ADP, AMP, or diadenosine oligophosphates APnA, where n is any number from 2-6). Additionally adenosine nucleotides includes analogs based on nucleotides comprising an adenine base. As used herein, an "analog" is a structural derivative of a parent compound (e.g., an adenosine nucleotide) that differs from it by one or more elements. In particular, 2871 receptor protein has been found to bind to AP6A, AMP, AP5A, AP4A, and ADP-ribose, as well as other adenine based nucleotides (referred to herein as adenosine nucleotides).

"AP6A" refers to di-adenosine hexaphosphate, which has the following structure:

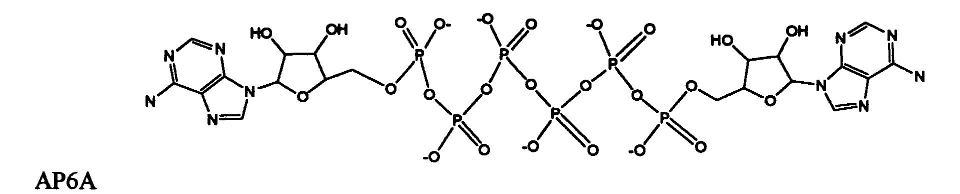

"AMP" refers to adenosine monophosphate, which has the following structure:

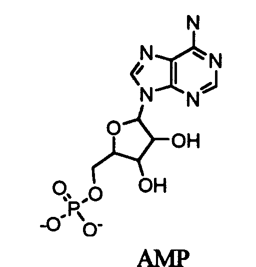

"AP5A" refers to di-adenosine pentaphosphate, which has the following structure:

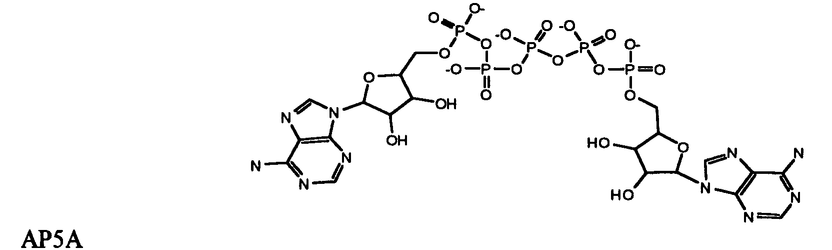

"AP4A" refers to di-adenosine tetraphosphate, which has the following 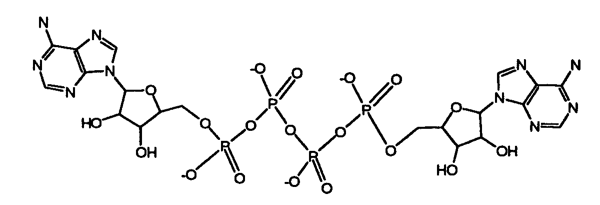 structure:
AP4A

"ADP-ribose" refers to adenosine di-phosphate-ribose, which has the following structure:

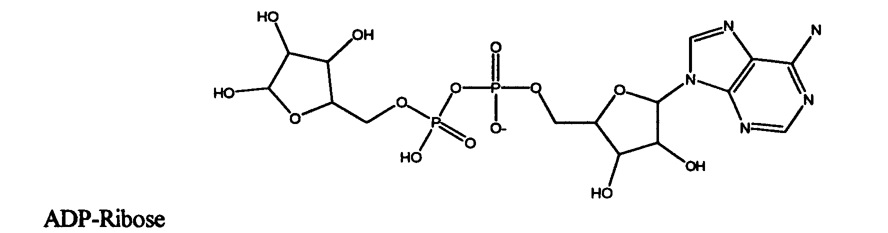

Fragments of the 2871 receptor protein are also useful in the methods of the present invention. Fragments can be derived from the naturally occurring 2871 receptor protein or from variants of the 2871 receptor protein as described herein. As used herein, a fragment comprises at least 12 contiguous amino acids. Fragments typically retain one or more of the functional or biological activities of the 2871 receptor protein, for example the ability to bind to a G-protein or ligand, as well as fragments that can be used as an immunogen to generate receptor antibodies. As used herein, "ligand binding fragment" is a fragment of naturally occurring 2871 receptor protein or of a variant of the 2871 receptor protein which has the ability to bind a 2871 receptor protein ligand (e.g., AP6A).

Useful fragments (peptides which are, for example, 12, 15, 20, 30, 35, 36, 37, 38, 39, 40, 50, 100 or more amino acids in length) can comprise a biologically active domain or motif, e.g., an extracellular domain, one or more of the seven transmembrane domains, G-protein binding domain, or GPCR signature domain. For example, useful fragments can include, but are not limited to: (i) soluble polypeptides comprising the entire N-terminal extracellular domain from about amino acid 1 to about amino acid 42 of SEQ ID NO:1; (ii) polypeptides comprising the C-terminal intracellular domain from about amino acid 319 to about amino acid 358 of SEQ ID NO:1; (iii) polypeptides comprising the entire 7-transmembrane domain from about amino acid 43 to amino acid 318; or any combination of i, ii, and/or iii or portions thereof (e.g., a polypeptide comprising the entire N-terminal extracellular domain, the entire 7-transmembrane domain, and a portion of the C-terminal domain).

Fragments can be discrete (not fused to other amino acids or polypeptides) or can be within a larger polypeptide. Further, several fragments can be comprised within a single larger polypeptide. In one embodiment a fragment designed for expression in a host can have heterologous pre- and propolypeptide regions fused to the amino terminus of the receptor fragment and an additional region fused to the carboxyl terminus of the fragment.

Chimeric or fusion proteins of the 2871 receptor protein are also useful in conjunction with the methods of the present invention. These comprise a receptor protein operatively linked to a heterologous protein having an amino acid sequence not substantially homologous to the receptor protein. "Operatively linked" indicates that the receptor protein and the heterologous protein are fused in-frame. The heterologous protein can be fused to the N-terminus or C-terminus of the receptor protein.

In one embodiment the fusion protein does not affect receptor function *per se.* For example, the fusion protein can be a GST-fusion protein in which the receptor sequences are fused to the C-terminus of the GST sequences. Other types of fusion proteins include, but are not limited to, enzymatic fusion proteins, for example beta-galactosidase fusions, yeast two-hybrid GAL fusions, poly-His fusions and Ig fusions. Such fusion proteins, particularly poly-His fusions, can facilitate the purification of recombinant receptor protein. In certain host cells (e.g., mammalian host cells), expression and/or secretion of a protein can be increased by using a heterologous signal sequence. Therefore, in another embodiment, the fusion protein contains a heterologous signal sequence at its N-terminus. Preferred fusion proteins of the invention include myc-tagged 2871 receptor proteins, and GFP-tagged 2871 receptor proteins.

European Patent Application Number 0 464 533 discloses fusion proteins comprising various portions of immunoglobin constant regions. The Fc is useful in therapy and diagnosis and thus results, for example, in improved pharmacokinetic properties (European Patent Application Number 0 232 262). In drug discovery, for example, human proteins have been fused with Fc portions for the purpose of high-throughput screening assays to identify antagonists. Bennett *et al*., *Journal of Molecular Recognition 8*:52-58 (1995) and Johanson *et al*., *The Journal of Biological Chemistry 270*,*16*:9459-9471 (1995). Thus, soluble fusion proteins containing a receptor polypeptide and various portions of the constant regions of heavy or light chains of immunoglobulins of various subclass (IgG, IgM, IgA, IgE) can be used. Preferred as immunoglobulin is the constant part of the heavy chain of human IgG, particularly IgG1, where fusion takes place at the hinge region. For some uses it is desirable to remove the Fc after the fusion protein has been used for its intended purpose, for example when the fusion protein is to be used as antigen for immunizations. The Fc part can be removed in a simple way by a cleavage sequence which is also incorporated and can be cleaved with factor Xa.

A chimeric or fusion protein can be produced by standard recombinant DNA techniques. For example, DNA fragments coding for the different protein sequences are ligated together in-frame in accordance with conventional techniques. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed and re-amplified to generate a chimeric gene sequence (see Ausubel *et al*., *Current Protocols in Molecular Biology,* 1992). Moreover, many expression vectors are commercially available that already encode a fusion moiety (e.g., a GST protein). A receptor protein-encoding nucleic acid can be cloned into such an expression vector such that the fusion moiety is linked in-frame to the receptor protein.

Another form of fusion protein is one that directly affects receptor functions. Accordingly, a receptor polypeptide encompassed by the present invention in which one or more of the receptor domains (e.g., the N-terminal extracellular tail, or the C-terminal intracellular tail) has been replaced by homologous domains from another G-protein coupled receptor or other type of receptor. Accordingly, various permutations are possible. Thus, chimeric receptors can be formed in which one or more of the native domains or subregions has been replaced. Methods and compositions for preparation and testing of chimeric G-protein coupled receptors are readily available and are known in the art (See, e.g., Howl, J. et al., FASEB J. 1997 Jun,11(7):582-90.; Gomeza, J. et al., J Biol Chem. 1996 Jan 26;271(4):2199-205.; and Sledziewski et al., U.S. Pat. No. 5,284,746.)

A composition comprising a 2871 receptor protein or functional fragment thereof (e.g., ligand-binding fragment) can be used in the methods and assays of the invention. A composition comprising a 2871 receptor protein or functional fragment thereof can include chimeric and/or fusion 2871 receptor proteins. Compositions suitable for use in the methods include, for example, cells which naturally express a 2871 receptor protein or functional fragment thereof or a 2871 receptor fusion protein or functional fragment thereof For example, cells that naturally express a 2871 receptor protein include hematopoeitic cells. Hematopoeitic cells include, red blood cells, macrophages, platelets, polymorphonuclear leucocytes, lymphocytes and CD34⁺ cells, including megakaryocytes. Additional cells which express a 2871 receptor protein include T-helper cells, glial cells, epithelial cells, and cells isolated from pancreatic, cardiac, prostate, placenta, uterus, testis, tonsil, thymus, and brain tissue may be utilized. Still further, tumorigenic cells which express 2871 receptor protein include breast tumor cells, ovary tumor cells, colon tumor cells, and lung tumor cells. Lung tumor cells can include NCI-H125 lung adenosquamous carcinoma cells. Additionally, cells that have been altered to express 2871 receptor protein or a functional fragment thereof can be used. Recombinant cells comprising an exogenous nucleic acid sequence which encodes a 2871 receptor protein or functional fragment thereof or a 2871 receptor protein or functional fragment thereof are also suitable for use in the methods.

Compositions comprising a 2871 receptor protein or functional fragment thereof (e.g., ligand-binding fragment) suitable for use in the methods also include, membrane preparations which comprise a 2871 receptor protein or functional fragment thereof, or a 2871 receptor fusion protein or functional fragment thereof. Such membrane preparations can contain natural (e.g., plasma membrane) or synthetic membranes. Preferably, the membrane preparation is a membrane fraction of a cell that expresses a 2871 receptor protein or a functional fragment thereof In one embodiment, paramagnetic proteoliposomes which contain a 2871 receptor protein, a 2871 receptor fusion protein or a functional fragment thereof can be used (see, for example, Mirzabekov, T. *et al*, *Nature Biotechnology*, *18*:649-654 (2000)).

Further, compositions comprising a 2871 receptor protein or functional fragment thereof (e.g., ligand-binding fragment) receptor polypeptides can include isolated 2871 receptor protein or a functional fragment thereof (e.g., ligand-binding fragment).

As used herein, a protein or polypeptide is said to be "isolated" or "purified" when it is substantially free of cellular material when it is isolated from recombinant and non-recombinant cells, or free of chemical precursors or other chemicals when it is chemically synthesized. A polypeptide, however, can be joined to another polypeptide with which it is not normally associated in a cell and still be considered "isolated" or "purified." It is understood, however, that preparations in which the polypeptide is not purified to homogeneity are useful and considered to contain an isolated form of the polypeptide. The critical feature is that the preparation allows for the desired function of the polypeptide, even in the presence of considerable amounts of other components.

The isolated 2871 receptor protein can be purified from cells that naturally express it, such as from hematopoeitic cells. Hematopoeitic cells include, red blood cells, macrophages, platelets, polymorphonuclear leucocytes, lymphocytes and CD34⁺ cells, including megakaryocytes. Additional cells which express a 2871 receptor protein include T-helper cells, glial cells, epithelial cells, and cells isolated from pancreatic, cardiac, prostate, placenta, uterus, testis, tonsil, thymus, and brain tissue may be utilized. Still further, tumorigenic cells which express 2871 receptor protein include breast tumor cells, ovary tumor cells, colon tumor cells, and lung tumor cells. Lung tumor cells can include NCI-H125 lung adenosquamous carcinoma cells. Additionally, cells that have been altered to express 2871 receptor protein (recombinant), or it can be synthesized using known protein synthesis methods.

Still further, cells expressing 2871 receptor protein such as stable or transient transfectants such as those described in the Examples herein or other suitable cells (e.g., baculovirus infected Sf9 cells), can be used for production of 2871 receptor protein, 2871 receptor fusion protein, or functional fragment thereof (e.g., ligand-binding fragment) by recombinant DNA techniques. For example, a nucleic acid molecule encoding the receptor polypeptide is cloned into an expression vector, the expression vector introduced into a host cell and the protein expressed in the host cell. The protein can then be isolated from the cells by an appropriate purification scheme using standard protein purification techniques.

In one embodiment, a human 2871 receptor cDNA is transfected into suitable cells (e.g., CHO or HEK293 cells). The human 2871 receptor cDNA (e.g., SEQ ID NO:2) is approximately 1489 nucleotides in length and encodes a full length 2871 receptor protein. The term "2871 polynucleotide" or "2871 nucleic acid" refers to the cDNA sequence shown in SEQ ID NO:2, or a sequence encoding the amino acid sequence of SEQ ID NO: 1. The term "receptor polynucleotide" or "receptor nucleic acid" further includes polynucleotides encoding fragments of the 2871 receptor protein.

"Receptor activity" or "functional activity" or "biological activity of the receptor" refers to the metabolic or physiologic function of 2871 receptor protein including similar activities or improved activities or these activities with decreased undesirable side-effects. Also included are antigenic and immunogenic activities of 2871 receptor protein. Particularly preferred activities of 2871 receptor, as well as polypeptides, nucleic acids, their uses and methods of the invention are described herein in further detail.

As used interchangeably herein a "2871 receptor protein activity", "biological activity of 2871 receptor" or "functional activity of 2871 receptor", refers to an activity exerted by a 2871 protein, polypeptide or nucleic acid molecule on a 2871 responsive cell as determined *in vivo,* or *in vitro,* according to standard techniques. In one embodiment, a 2871 receptor protein activity is a direct activity, such as an association with a target protein, preferably a 2871 target molecule (e.g., a G-protein alpha subunit or a 2871 ligand). In another embodiment, a 2871 receptor protein activity is an indirect activity, such as inhibiting the synthesis or activity of a second protein (e.g., a protein of a signal transduction pathway). In a preferred embodiment, a 2871 receptor protein activity is at least one or more of the following activities: (i) interaction of a 2871 protein in the plasma membrane with a protein or other organic molecule secreted from the same cell which expresses the 2871 protein molecule (e.g., a 2871 ligand); (ii) interaction of a 2871 protein in the plasma membrane with a protein or other organic molecule secreted from a different cell from that which contains the 2871 protein molecule (e.g., a 2871 ligand); (iii) complex formation between a 2871 protein and a secreted peptide, polypeptide, or small molecule; (iv) interaction of a 2871 protein with a target molecule in the extracellular milieu (e.g., a soluble target molecule); (v) interaction of the 2871 protein with an intracellular target molecule (e.g., interaction with an internalized or endocytosed ligand); and (vi) complex formation with one, two, or more, intracellular target molecules.

In yet another preferred embodiment, a 2871 receptor protein activity is at least one or more of the following activities: (i) the ability to modulate, for example, agonize or antagonize a signal transduction pathway (e.g., a 2871-dependent pathway); (ii) the ability to modulate cytokine production and/or secretion (e.g., production and/or secretion of a proinflammatory cytokine); (iii) the ability to modulate lymphokine production and/or secretion; (iv) the ability to modulate brain function; (v) the ability to modulate production of adhesion molecules and/or cellular adhesion; (vi) the ability to modulate expression or activity of nuclear transcription factors; (vii) the ability to modulate expression of IL-4, IL-5, or of other cytokines involved in T-cell function; (viii) the ability to modulate cell (e.g., a cell which has the ability to express 2871 receptor protein, e.g., as described herein) proliferation, development or differentiation (e.g., helper T-cell differentiation to Th1 versus Th2 cells); (ix) the ability to modulate cell proliferation, development or differentiation of bone marrow and/or megakaryocyte precursor cells; (x) the ability to modulate cellular immune responses; (xi) the ability to modulate cytokine-mediated proinflammatory actions (e.g., inhibiting acute phase protein synthesis by hepatocytes, fever, and/or prostaglandin synthesis, for example PGE₂ synthesis); (xii) the ability to promote and/or potentiate wound healing; and, (xiii) the ability to modulate cellular proliferation.

In yet another preferred embodiment, a 2871 receptor protein activity includes the ability to modulate the differentiation, development, proliferation, and the production of hematopoietic cells in the lymphoid cell (e.g., lymphocyte, B cell, T cell), and myeloid cell (e.g., megakaryocyte (e.g., platelet), granulocyte (e.g., neutrophil, eosinophil, basophil, monocyte), and erythroid cell (e.g., erythrocyte)) lineages. These include CD34⁺ stem cells that give rise to cells of all three lineages, and to the subsequently produced progenitor cells in the developmental pathway that gives rise to the three fully differentiated cell types.

### Identification of Ligands, Inhibitors or Promoters of Receptor Function

As used herein, a ligand is a substance which binds to a receptor protein. A ligand of a selected 2871 receptor protein is a substance which binds to the selected 2871 receptor protein. In a preferred embodiment, ligand binding of a 2871 receptor protein occurs with high affinity (e.g., with an affinity of at least about 1 x 10⁷ M⁻¹, or with an affinity that is at least two-fold, 50-fold, 100-fold, or greater than its affinity for binding to a non-specific protein (e.g., BSA, casein) other than 2871 receptor protein). The term ligand refers to substances including, but not limited to, a natural ligand, whether isolated and/or purified, synthetic, and/or recombinant, a homolog of a natural ligand (e.g., from another mammal), antibodies, portions of such molecules, and other substances which bind receptor. A natural ligand of a selected mammalian receptor can bind to the receptor under physiological conditions, and is of a mammalian origin which is the same as that of the 2871 receptor protein. The term ligand encompasses substances which are inhibitors or promoters of receptor activity, as well as substances which bind receptor, but lack inhibitor or promoter activity.

As used herein, an inhibitor is a substance which inhibits at least one function characteristic of a 2871 receptor protein, such as a binding activity (e.g., ligand binding, promoter binding), a signalling activity (e.g., activation of a mammalian G protein, induction of rapid and transient increase in the concentration of cytosolic free calcium [Ca.²⁺]), and/or cellular response function (e.g., stimulation of chemotaxis, actin polymerization, shape change, exocytosis or apoptosis). The term inhibitor refers to substances including antagonists which bind receptor (e.g., an antibody, a mutant of a natural ligand, other competitive inhibitors (e.g., small organic molecules) of ligand binding), and substances which inhibit receptor function without binding thereto (e.g., an anti-idiotypic antibody).

As used herein, a promoter is a substance which promotes (induces or enhances) at least one function characteristic of a 2871 receptor protein, such as a binding activity (e.g., ligand, inhibitor and/or promoter binding), a signalling activity (e.g., activation of a mammalian G protein, induction of rapid and transient increase in the concentration of cytosolic free calcium [Ca²⁺]), and/or a cellular response function (e.g., stimulation of chemotaxis, actin polymerization, shape change, exocytosis or apoptosis). The term promoter refers to substances including agonists which bind receptor (e.g., an antibody, a homolog of a natural ligand from another species), and substances which promote receptor function without binding thereto (e.g., by activating an associated protein). In a preferred embodiment, the agonist is other than a homolog of a natural ligand.

According to the methods of the present invention, compounds can be individually screened or one or more compounds can be tested simultaneously according to the methods herein. Where a mixture of compounds is tested, the compounds selected by the processes described can be separated (as appropriate) and identified by suitable methods (e.g., PCR, sequencing, chromatography). The presence of one or more compounds (e.g., a ligand, inhibitor, promoter) in a test sample can also be determined according to these methods.

Large combinatorial libraries of compounds (e.g., lipids, organic compounds (e.g., small organic compounds), recombinant or synthetic peptides, "peptoids", nucleotides, nucleosides, nucleic acids, or analogs thereof) produced by combinatorial chemical synthesis or other methods can be tested (see e.g., Zuckerman, R.N. et al., J. Med. Chem., 37: 2678-2685 (1994) and references cited therein; see also, Ohlmeyer, M. H. J. et al., Proc. Natl. Acad. Sci. USA 90:10922-10926 (1993) and DeWitt, S. H. et al., Proc. Natl. Acad. Sci. USA 90:6909-6913 (1993), relating to tagged compounds; Rutter, W. J. et al. U.S. Pat. No. 5,010,175; Huebner, V .D. et al., U.S. Pat. No. 5,182,366; and Geysen, H. M., U.S. Pat. No. 4,833,092). Where compounds selected from a combinatorial library by the present method carry unique tags, identification of individual compounds by chromatographic methods is possible.

Other sources of potential ligands, inhibitors and/or promoters of 2871 receptor proteins include, but are not limited to, variants of 2871 receptor protein ligands, including naturally occurring, synthetic or recombinant variants of adenosine nucleotide substances such as other adenine-based nucleotides or analogs, variants thereof, other inhibitors and/or promoters (e.g., anti-2871 receptor protein antibodies, antagonists, agonists), other G-protein coupled receptor ligands, inhibitors and/or promoters (e.g., antagonists or agonists), and soluble portions of a 2871 receptor receptor, such as a suitable receptor peptide or analog which can inhibit receptor function (see e.g., Murphy, R. B., WO 94/05695).

The assays described herein can be used, alone or in combination with each other or other suitable methods, to identify ligands, inhibitors or promoters of a 2871 receptor protein or variant thereof. The *in vitro* methods of the present invention can be adapted for high-throughput screening in which large numbers of samples are processed (e.g., a 96 well format). Host cells comprising a nucleic acid of the present invention and expressing recombinant 2871 receptor (e.g., human 2871 receptor protein) at levels suitable for high-throughput screening can be used, and thus, are particularly valuable in the identification and/or isolation of ligands, inhibitors and promoters of 2871 receptor proteins. Expression of receptor can be monitored in a variety of ways. For instance, expression can be monitored using antibodies of the present invention which bind receptor or a portion thereof. Also, commercially available antibodies can be used to detect expression of an antigen- or epitope-tagged fusion protein comprising a receptor protein or polypeptide (e.g., myc- or FLAG- tagged receptors), and cells expressing the desired level can be selected. Binding Assays

The isolated and/or recombinant 2871 receptor proteins, portions thereof, or suitable fusion proteins of the present invention, can be used in a method to select and identify agents which bind to a (one or more) 2871 receptor protein, such as human 2871 receptor protein, and which are ligands, or potential inhibitors or promoters of receptor activity. Agents selected by the method, including ligands, inhibitors or promoters, can be further assessed for an inhibitory or stimulatory effect on receptor function and/or for therapeutic utility.

In one embodiment, an agent which binds to an active, isolated and/or recombinant 2871 receptor protein or polypeptide is identified by the method. In this embodiment, the receptor protein or polypeptide used has at least one property, activity or function characteristic of a 2871 receptor protein (as defined herein), such as a binding activity (e.g., ligand, inhibitor and/or promoter binding), a signalling activity (e.g., activation of a mammalian G protein, induction of rapid and transient increase in the concentration of cytosolic free calcium [Ca²⁺]), cellular response function (e.g., stimulation of chemotaxis, actin polymerization, shape change, exocytosis or apoptosis), and/or an hematological or immunological property (e.g., stimulation of blood and/or inflammation processes, and/or mediation of blood and/or inflammatory disorders) as described herein. In a preferred embodiment, the isolated and/or recombinant 2871 receptor protein or fragment thereof has ligand binding function, and more preferably binds a natural ligand of the receptor. In a particularly preferred embodiment, the isolated and/or recombinant protein is a human 2871 receptor protein encoded by the nucleic acid of SEQ ID NO:2.

For example, a composition comprising an isolated and/or recombinant 2871 receptor protein or fragment thereof can be maintained under conditions suitable for binding, the receptor can be contacted with an agent (e.g., a composition comprising one or more agents) to be tested, and binding is detected or measured. In one embodiment, a receptor protein can be expressed in cells stably or transiently transfected with a construct comprising a nucleic acid sequence which encodes a receptor of the present invention. The cells can be maintained under conditions appropriate for expression of 2871 receptor protein. The cells are contacted with an agent under conditions suitable for binding (e.g., in a suitable binding buffer), and binding can be detected by standard techniques. For example, the extent of binding can be determined relative to a suitable control (e.g., compared with background determined in the absence of agent, compared with binding of a second agent (i.e., a standard), compared with binding of the agent to untransfected cells). Optionally, a cellular fraction, such as a membrane fraction, containing receptor can be used in lieu of whole cells. Additionally, membrane preparations, including microsome preparations, and synethetic lipid-bilayer compositions containing 2871 receptor proteins or ligand binding fragments thereof may be used in conjunction with the methods of the present invention.

Binding or complex formation can be detected directly or indirectly. In one embodiment, the agent can be labeled with a suitable label (e.g., fluorescent label, chemiluminescent label, chromoluminescent label, isotope label, enzyme label), and binding can be determined by detection of the label. Specificity of binding can be assessed by competition or displacement, for example, using unlabeled agent or a ligand (e.g., AP6A or AMP) as competitor.

Ligands of the mammalian receptor, including natural ligands from the same mammalian species or from another species, can be identified in this manner. The binding activity of a promoter or inhibitor which binds receptor can also be assessed using such a ligand binding assay.

Binding inhibition assays can also be used to identify ligands, as well as inhibitors and promoters which bind receptor and modulate binding of another agent such as a ligand. For example, a binding assay can be conducted in which a reduction in the binding of a first agent (in the absence of a second agent), as compared with binding of the first agent in the presence of the second test agent, is detected or measured. The receptor can be contacted with the first and second agents simultaneously, or one after the other, in either order. A reduction in the extent of binding of the first agent in the presence of the second test agent, is indicative of inhibition of binding by the second agent. For example, binding of the first agent could be decreased or abolished.

In one embodiment, direct inhibition of the binding of a first agent (e.g., an adenosine nucleotide such as AP6A or AMP) to a 2871 receptor protein by a second test agent is monitored. For example, the ability of an agent to inhibit the binding of ³² P-labeled AP6A to 2871 receptor protein can be monitored. Such an assay can be conducted using whole cells (e.g., a suitable cell line containing nucleic acid encoding a human 2871 receptor protein), or a membrane fraction from said cells, for instance.

One candidate compound is a soluble full-length 2871 receptor or a 2871 receptor fragment that competes for ligand binding. Other candidate compounds include mutant 2871 protein receptors or appropriate fragments containing mutations that affect receptor function and thus compete for ligand. Accordingly, a fragment that competes for ligand, for example with a higher affinity, or a fragment that binds ligand but does not allow release, is encompassed by the invention.

Other methods of identifying the presence of an agent(s) which binds a receptor are available, such as methods which monitor events which are triggered by receptor binding, including signalling function and/or stimulation of a cellular response.

It will be understood that the inhibitory effect of antibodies of the present invention can be assessed in a binding inhibition assay. Competition between antibodies for receptor binding can also be assessed in the method in which the first agent in the assay is another antibody, under conditions suitable for antibody binding.

Ligands, receptor-binding inhibitors and promoters, which are identified in this manner, can be further assessed to determine whether, subsequent to binding, they act to inhibit or activate other functions of 2871 receptor proteins and/or to assess their therapeutic utility.

To perform cell free screening assays, it can be desirable to immobilize either the 2871 receptor protein, or fragment, or its target molecule (e.g., an adenosine nucleotide) to facilitate separation of complexes from uncomplexed forms of one or both of the proteins, as well as to accommodate automation of the assay.

Techniques for immobilizing proteins on matrices can be used in the drug screening assays. In one embodiment, a fusion protein can be provided which adds a domain that allows the protein to be bound to a matrix. For example, glutathione-S-transferase/2871 fusion proteins can be adsorbed onto glutathione sepharose beads (Sigma Chemical, St. Louis, MO) or glutathione derivatized microtitre plates, which are then combined with the cell lysates (e.g., ³⁵S-labeled) and the candidate compound, and the mixture incubated under conditions conducive to complex formation (e.g., at physiological conditions for salt and pH). Following incubation, the beads are washed to remove any unbound label, and the matrix immobilized and radiolabel determined directly, or in the supernatant after the complexes are dissociated. Alternatively, the complexes can be dissociated from the matrix, separated by SDS-PAGE, and the level of receptor-binding protein found in the bead fraction quantitated from the gel using standard electrophoretic techniques. For example, either the polypeptide or its target molecule can be immobilized utilizing conjugation of biotin and streptavidin using techniques well known in the art. Alternatively, antibodies reactive with the protein but which do not interfere with binding of the protein to its target molecule can be derivatized to the wells of the plate, and the protein trapped in the wells by antibody conjugation. Preparations of a receptor-binding protein and a candidate compound are incubated in the receptor protein-presenting wells and the amount of complex trapped in the well can be quantitated. Methods for detecting such complexes, in addition to those described above for the GST-immobilized complexes, include immunodetection of complexes using antibodies reactive with the receptor protein target molecule, or which are reactive with receptor protein and compete with the target molecule; as well as enzyme-linked assays which rely on detecting an enzymatic activity associated with the target molecule.

### Signaling Assays

The binding of a G protein-coupled receptor (e.g., by an agonist) can result in signaling by the receptor, and stimulation of the activity of G protein. The induction of signaling function by an agent can be monitored using any suitable method. For example, G protein activity, such as hydrolysis of GTP to GDP, or later signaling events triggered by receptor binding, such as induction of rapid and transient increase in the concentration of intracellular (cytosolic) free calcium [Ca²⁺ ] can be assayed by methods known in the art or other suitable methods (Examples; see also, Neote, K. et al., Cell, 72: 415-425 1993); Van Riper et al., J. Exp. Med., 177: 851-856 (1993); Dahinden, C.A. et al., J. Exp. Med., 179: 751-756 (1994)). Additionally, changes in the intracellular concentration of cAMP levels can be measured by methods known in the art or other suitable methods (Examples; see also, Unson, et al., (1996) Proc. Natl. Acad. Sci. U. S. A. 93: 310-315; and Cypress et al., J Biol Chem. 1999 Jul 2;274(27):19455-64). Still further, changes in intracellular pH as a result of signaling can be measured by standard methods known in the art (see Examples).

The functional assay of Sledziewski et al. using hybrid G protein coupled receptors can also be used to identify a ligand or promoter by its ability to activate a hybrid G protein or to identify an inhibitor by its ability to inhibit such activation (Sledziewski et al., U.S. Pat. No. 5,284,746, the teachings of which are incorporated herein by reference). In one embodiment, a biological response of the host cell (triggered by binding to receptor or hybrid receptor) can be monitored, detection of the response being indicative of the presence of ligand in the test sample. For example, a method of detecting the presence of a ligand in a test sample is described, wherein the ligand is an agent which is capable of being bound by the ligand-binding domain of a receptor. In one embodiment of the method, yeast host cells are transformed with a DNA construct capable of directing the expression of a biologically active hybrid G protein-coupled receptor (i.e., a fusion protein). The hybrid receptor comprises a mammalian G protein-coupled receptor having at least one domain other than the ligand-binding domain replaced with a corresponding domain of a yeast G protein-coupled receptor, such as a STE2 gene product. The yeast host cells containing the construct are maintained under conditions in which the hybrid receptor is expressed, and the cells are contacted with a test sample under conditions suitable to permit binding of ligand to the hybrid receptor. A biological response of the host cell (triggered by binding to hybrid receptor) is monitored, detection of the response being indicative of a signalling function. For instance, binding to a hybrid receptor derived from STE2 gene product can lead to induction of the BAR1 promoter. Induction of the promoter can be measured by means of a reporter gene (e.g., .beta.-gal), which is linked to the BAR1 promoter and introduced into host cells on a second construct. Expression of the reporter gene can be detected by an in vitro enzyme assay on cell lysates or by the presence of blue colonies on plates containing an indicator (e.g., X-gal) in the medium, for example.

In another embodiment, the assay can be used to identify potential inhibitors of receptor function. The inhibitory activity of an agent can be determined using a ligand or promoter in the assay, and assessing the ability of the test agent to inhibit the activity induced by ligand or promoter.

Variants of known ligands can also be screened for reduced ability (decreased ability or no ability) to stimulate activity of a coupled G protein. In this embodiment, although the agent has ligand binding activity (as determined by another method), engagement of the receptor does not trigger or only weakly triggers activity of a coupled G protein. Such agents are potential antagonists, and can be further assessed for modulatory activity.

### Chemotaxis and Other Assays of Cellular Responses

Chemotaxis assays can also be used to assess receptor function. These assays are based on the functional migration of cells *in vitro* or *in vivo* induced by an agent, and can be used to assess the binding and/or effect on chemotaxis of ligands, inhibitors, or promoters. Springer et al. describe a transendothelial lymphocyte chemotaxis assay (Springer et al., WO 94/20142, published Sep. 15, 1994, the teachings of which are incorporated herein by reference; see also Berman et al., Immunol Invest., 17: 625-677 (1988)). Migration across endothelium into collagen gels has also been described (Kavanaugh et al., J. Immunol, 146: 4149-4156 (1991)).

Generally, chemotaxis assays monitor the directional movement or migration of a suitable cell capable of chemotaxis, such as a leukocyte (e.g., T lymphocytes, NK cells, monocytes), stable transfectants of Jurkat cells, mouse L1-2 pre-B cells or of other suitable host cells, for example, into or through a barrier (e.g., endothelium, a filter), toward increased levels of an agent, from a first surface of the barrier toward an opposite second surface. Membranes or filters provide convenient barriers, such that the directional movement or migration of a suitable cell into or through a filter, toward increased levels of an agent, from a first surface of the filter toward an opposite second surface of the filter, is monitored. In some assays, the membrane is coated with a substance to facilitate adhesion, such as ICAM-1, fibronectin or collagen, followed by adhesion monitoring. In some assay, shape change (e.g., actin polymerization) can be measured

For example, one can detect or measure the migration of cells in a suitable container (a containing means), from a first chamber into or through a microporous membrane into a second chamber which contains an agent to be tested, and which is divided from the first chamber by the membrane. A suitable membrane, having a suitable pore size for monitoring specific migration in response to the agent, including, for example, nitrocellulose, polycarbonate, is selected. For example, pore sizes of about 3-8 microns, and preferably about 5-8 microns can be used. Pore size can be uniform on a filter or within a range of suitable pore sizes.

To assess migration, the distance of migration into the filter, the number of cells crossing the filter that remain adherent to the second surface of the filter, and/or the number of cells that accumulate in the second chamber can be determined using standard techniques (e.g., by microscopy). In one embodiment, the cells are labeled with a detectable label (e.g., radioisotope, fluorescent label, antigen or epitope label), and migration can be assessed by determining the presence of the label adherent to the membrane and/or present in the second chamber using an appropriate method (e.g., by detecting radioactivity, fluorescence, immunoassay). The extent of migration induced by an agent can be determined relative to a suitable control (e.g., compared to background migration determined in the absence of the agent, to the extent of migration induced by a second agent (i.e., a standard), compared with migration of untransfected cells induced by the agent).

To assess shape change (e.g., actin polymerization),methods known in the art can be utilized, or other suitable methods. For example, actin microfilaments; cells can be fixed with 4% formaldehyde, permeabilized with 0.2% Triton-X 100, and stained with TRITC-phalloidin (0.05 µg/ml; Sigma) to visualize actin filaments.

Chambers can be formed from various solids, such as plastic, glass, polypropylene, polystyrene, etc. Membranes which are detachable from the chambers, such as a Biocoat (Collaborative Biomedical Products) or Transwell (Costar, Cambridge, Mass.) culture insert, facilitate counting adherent cells. In the container, the filter can be situated so as to be in contact with fluid containing cells in the first chamber, and the fluid in the second chamber. Other than the test agent or additional ligand, inhibitor, or promoter present for the purpose of the assay, the fluid on either side of the membrane is preferably the same or substantially similar. The fluid in the chambers can comprise protein solutions (e.g., bovine serum albumin, fetal calf serum, human serum albumin) which may act to increase stability and inhibit nonspecific binding of cells, and/or culture media.

In one embodiment, transendothelial migration is assessed. In addition to lower background (signal to noise ratio), transendothelial migration models *in vivo* conditions in which leukocytes emigrate from blood vessels toward chemoattractants present in the tissues at sites of inflammation by crossing the endothelial cell layer lining the vessel wall. In this embodiment, transmigration through an endothelial cell layer assessed. To prepare the cell layer, endothelial cells can be cultured on a microporous filter or membrane, optionally coated with a substance such as collagen, fibronectin, or other extracellular matrix proteins, to facilitate the attachment of endothelial cells. Preferably, endothelial cells are cultured until a confluent monolayer is formed. A variety of mammalian endothelial cells can are available for monolayer formation, including for example, vein, artery or microvascular endothelium, such as human umbilical vein endothelial cells (Clonetics Corp, San Diego, Calif.) or a suitable cell line, such as the ECV 304 cell line used (European Collection of Animal Cell Cultures, Porton Down, Salisbury, U.K.). To assay chemotaxis in response to a particular mammalian receptor, endothelial cells of the same mammal are preferred; however endothelial cells from a heterologous mammalian species or genus can also be used.

Generally, the assay is performed by detecting the directional migration of cells into or through a membrane or filter, in a direction toward increased levels of an agent, from a first surface of the filter toward an opposite second surface of the filter, wherein the filter contains an endothelial cell layer on a first surface. Directional migration occurs from the area adjacent to the first surface, into or through the membrane, towards an agent situated on the opposite side of the filter. The concentration of agent present in the area adjacent to the second surface is greater than that in the area adjacent to the first surface.

In one embodiment, a chemotaxis assay is used to test for ligand or promoter activity of an agent, a composition comprising cells capable of migration and expressing a 2871 receptor protein are placed in the first chamber, and a composition comprising the agent (one or more agents) to be tested is placed in the second chamber, preferably in the absence of other ligands or promoters capable of inducing chemotaxis of the cells in the first chamber (having chemoattractant function). However, one or more ligands or promoters having chemoattractant function may be present. The ability of an agent to induce chemotaxis of the cells expressing a 2871 receptor protein in this assay is indicative that the agent is a ligand or promoter of receptor function.

In one embodiment used to test for an inhibitor, a composition comprising cells capable of migration and expressing a 2871 receptor protein are placed in the first chamber. A composition comprising a ligand or promoter (i.e., one or more ligands or promoters) capable of inducing chemotaxis of the cells in the first chamber (having chemoattractant function) is placed in the second chamber. Before (preferably shortly before) the cells are placed in the first chamber, or simultaneously with the cells, a composition comprising the agent to be tested is placed, preferably, in the first chamber. The ability of an agent to inhibit ligand- or promoter-induced chemotaxis of the cells expressing a 2871 receptor protein in this assay is indicative that the agent is an inhibitor of receptor function (e.g., an inhibitor of cellular response function). A reduction in the extent of migration induced by the ligand or promoter in the presence of the test agent, is indicative of inhibitory activity. Separate binding studies (see above) can be performed to determine whether inhibition is a result of binding of the test agent to receptor or occurs via a different mechanism.

The effects of a ligand, inhibitor or promoter on the cellular response function of a 2871 receptor protein can be assessed by monitoring other cellular responses induced by active receptor, using suitable host cells containing receptor. Similarly, these assays can be used to determine the function of a receptor. For instance, exocytosis (e.g., degranulation of natural killer cells leading to release of one or more enzymes or other granule components, such as esterases (e.g., serine esterases), perforin, and/or granzymes), inflammatory mediator release (such as release of bioactive lipids such as leukotrienes (e.g., leukotriene C4) ), and respiratory burst, can be monitored by methods known in the art or other suitable methods. (See e.g., Taub, D. D. et al., J. Immunol., 155: 3877-3888 (1995) regarding assays for release of granule-derived serine esterases (the teachings of which are incorporated herein by reference) and Loetscher et al., J. Immunol., 156: 322-327 (1996) regarding assays for enzyme and granzyme release by NK cells and cytotoxic T lymphocytes (CTLs) (the teachings of which are incorporated herein by reference); Rot, A. et al., J. Exp. Med., 176: 1489-1495 (1992) regarding respiratory burst; Bischoff. S. C. et al., Eur. J. Immunol., 23: 761-767 (1993) and Baggliolini, M. and C. A. Dahinden, Immunology Today, 15: 127-133 (1994)).

In one embodiment, a ligand, inhibitor and/or promoter is identified by monitoring the release of an enzyme upon degranulation or exocytosis by a cell capable of this function. Cells containing a nucleic acid of the present invention, which encodes an active receptor protein capable of stimulating exocytosis or degranulation are maintained in a suitable medium under suitable conditions, whereby receptor is expressed and degranulation can be induced. The receptor is contacted with an agent to be tested, and enzyme release is assessed. The release of an enzyme into the medium can be detected or measured using a suitable assay, such as in an immunological assay, or biochemical assay for enzyme activity.

The medium can be assayed directly, by introducing components of the assay (e.g., substrate, co-factors, antibody) into the medium (e.g., before, simultaneous with or after the cells and agent are combined). Alternatively, the assay can be performed on medium which has been separated from the cells or further processed (e.g., fractionated) prior to assay. For example, convenient assays for are available for enzymes such serine esterases (see e.g., Taub, D. D. et al., J. Immunol., 155: 3877-3888 (1995) regarding release of granule-derived serine esterases).

Stimulation of degranulation by an agent can be indicative that the agent is a ligand or promoter of a 2871 receptor protein. In another embodiment, cells expressing receptor are combined with a ligand or promoter, and an agent to be tested is added before, after or simultaneous therewith, and degranulation is assessed. Inhibition of ligand- or promoter-induced degranulation is indicative that the agent is an inhibitor of 2871 receptor protein function.

Cell adherence can also monitored by methods known in the art or other suitable methods. Engagement of the receptors of a lymphocyte can cause integrin activation, and induction of adherence to adhesion molecules expressed in vasculature or the perivascular space. In one embodiment, a ligand, inhibitor and/or promoter is identified by monitoring cellular adherence by a cell capable of adhesion. For example, an agent to be tested can be combined with (a) cells expressing receptor (preferably non-adherent cells which when transfected with receptor aquire adhesive ability), (b) a composition comprising a suitable adhesion molecule (e.g., a substrate such as a culture well coated with an adhesion molecule, such as fibronectin), and (c) a ligand or promoter (e.g., agonist), and maintained under conditions suitable for ligand- or promoter-induced adhesion. Labeling of cells with a fluorescent dye provides a convenient means of detecting adherent cells. Nonadherent cells can be removed (e.g., by washing) and the number of adherent cells determined. The effect of the agent in inhibiting or enhancing ligand- or promoter-induced adhesion can be indicative of inhibitor or promoter activity, respectively. Agents active in the assay include inhibitors and promoters of binding, signalling, and/or cellular responses. In another embodiment, an agent to be tested can be combined with cells expressing receptor and a composition comprising a suitable adhesion molecule under conditions suitable for ligand- or promoter-induced adhesion, and adhesion is monitored. Increased adhesion relative to a suitable control is indicative of the presence of a ligand and/or promoter.

Still further, cellular response assays can be used to determine the effects of a ligand, inhibitor or promoter on the function of a 2871 receptor protein. For instance, effects can be assessed by monitoring cellular responses (e.g., cell differentiation, cell proliferation, and apoptosis (i.e., cell death) induced by active receptor using suitable host cells containing receptor. Such cellular responses can be monitored and assessed by methods known in the art or other suitable methods. For example, progenitor cells exposed to an agent in conjunction with ligand can be monitored for subsequent differentiation. Such monitoring is dependent on the type of cell/differentiation desired (e.g., hematopoietic differentiation from hematopoietic precursor cells to lymphocyte or platelets) to monitor, and compositions and methods are numerous and well known in the art. Still further, monitoring of cell proliferation or cell death in response to 2871 receptor ligand, inhibitor or promoter activity may be performed utilizing cells expressing 2871 receptor protein (e.g., cells described herein) according to methods well known in the art to assess cell numbers over time, and cell viability.

Binding and/or activating compounds can also be screened by using chimeric receptor proteins in which the N-terminal extracellular domain, the transmembrane domain or subregions, and the C-terminal intracellular domain can be replaced by heterologous domains. For example, a G-protein-binding region can be used that interacts with a different G-protein then that which is recognized by the native receptor. Accordingly, a different set of signal transduction components is available as an end-point assay for activation. Alternatively, the transmembrane portion can be replaced with a transmembrane portion specific to a host cell that is different from the host cell from which the extracellular domain and/or the G-protein-binding region are derived. This allows for assays to be performed in other than the specific host cell from which the receptor is derived. Alternatively, the extracellular domain could be replaced by a domain binding a different ligand, thus, enabling an assay for test compounds that interact with the heterologous extracellular domain but still cause signal transduction. Furthermore, chimeric 2871 receptor polypeptides are also useful in competition binding assays in methods designed to discover compounds that interact with regions of the receptor. Thus, a compound is exposed to a receptor polypeptide under conditions that allow the compound to bind or to otherwise interact with the polypeptide. Soluble receptor polypeptide is also added to the mixture. If the test compound interacts with the soluble chimeric receptor polypeptide, it decreases the amount of complex formed or activity from the receptor target. This type of assay is particularly useful in cases in which compounds are sought that interact with specific regions of the receptor. Thus, the soluble chimeric polypeptide that competes with the target receptor region is designed to contain peptide sequences corresponding to the region of interest.

### Antibodies

The invention also provides antibodies that selectively bind to the 2871 receptor protein and its variants and fragments. An antibody is considered to selectively bind, even if it also binds to other proteins that are not substantially homologous with the receptor protein. These other proteins share homology with a fragment or domain of the receptor protein. This conservation in specific regions gives rise to antibodies that bind to both proteins by virtue of the homologous sequence. In this case, it would be understood that antibody binding to the receptor protein is still selective.

"Antibodies" as used herein includes polyclonal and monoclonal antibodies. The terms polyclonal and monoclonal refer to the degree of homogeneity of an antibody preparation, and are not intended to be limited to particular methods of production. The term "antibody" as used herein also encompasses functional fragments of antibodies, including fragments of chimeric, human, humanized, deimmunized, primatized, veneered or single chain antibodies. Functional fragments including antigen-binding fragments, including, but not limited to Fv, Fab, Fab' and F(ab')₂ fragments are encompassed by the invention. Such fragments can be produced by enzymatic cleavage or by recombinant techniques. For example, papain or pepsin cleavage can generate Fab or F(ab')₂ fragments, respectively. Other proteases with the requisite substrate specificity can also be used to generate Fab or F(ab')₂ fragments. Antibodies can also be produced in a variety of truncated forms using antibody genes in which one or more stop codons has been introduced upstream of the natural stop site. For example, a chimeric gene encoding a F(ab')₂ heavy chain portion can be designed to include DNA sequences encoding the CH₁ domain and hinge region of the heavy chain.

Single chain antibodies, and chimeric, humanized deimmunized, or primatized (CDR-grafted), or veneered antibodies comprising portions derived from different species, and the like are also encompassed by the present invention and the term "antibody". The various portions of these antibodies can be joined together chemically by conventional techniques, or can be prepared as a contiguous protein using recombinant DNA technology. For example, nucleic acids encoding a chimeric or humanized chain can be expressed to produce a contiguous protein. See, e.g., Cabilly *et al*., U.S. Patent No. 4,816,567; Cabilly *et al*., European Patent No. 0,125,023 B1; Boss *et al*., U.S. Patent No. 4,816,397; Boss *et al*., European Patent No. 0,120,694 B1; Neuberger, M.S. *et al*., WO 86/01533; Neuberger, M.S. *et al*., European Patent No. 0,194,276 B1; Winter, U.S. Patent No. 5,225,539; Winter, European Patent No. 0,239,400 B1; Queen *et al*., European Patent No. 0 451 216 B1; and Padlan, E.A. *et al*., EP 0 519 596 A1. See also, Newman, R. *et al*., *BioTechnology*, *10:* 1455-1460 (1992), regarding primatized antibody, and Ladner *et al*., U.S. Patent No. 4,946,778 and Bird, R.E. *et al*., *Science, 242:* 423-426 (1988)) regarding single chain antibodies.

Humanized antibodies can be produced using synthetic or recombinant DNA technology using standard methods or other suitable techniques. Nucleic acid (e.g., cDNA) sequences coding for humanized variable regions can also be constructed using PCR mutagenesis methods to alter DNA sequences encoding a human or humanized chain, such as a DNA template from a previously humanized variable region (see e.g., Kamman, M., *et al*., *Nucl. Acids Res*., *17*: 5404 (1989)); Sato, K., *et al*., *Cancer Research*, *53:* 851-856 (1993); Daugherty, B.L. *et al*., *Nucleic Acids Res*., *19(9):* 2471-2476 (1991); and Lewis, A.P. and J.S. Crowe, *Gene, 101*: 297-302 (1991)). Using these or other suitable methods, variants can also be readily produced. In one embodiment, cloned variable regions can be mutated, and sequences encoding variants with the desired specificity can be selected (e.g., from a phage library; see e.g., Krebber *et al*., U.S. 5,514,548; Hoogenboom *et al*., WO 93/06213, published April 1, 1993). Humanized antibodies can be generated by replacing sequences of the Fv variable region which are not directly involved in antigen binding with equivalent sequences from human Fv variable regions. General methods for generating humanized antibodies are provided by Morrison (1985) *Science* 229:1202-1207, by Oi *et al.* (1986) *BioTechniques* 4:214, and by Queen *et al.* US patent Nos. 5,585,089, 5,693,761 and 5,693,762, the contents of all of which are hereby incorporated by reference. Those methods include isolating, manipulating, and expressing the nucleic acid sequences that encode all or part of immunoglobulin Fv variable regions from at least one of a heavy or light chain. Sources of such nucleic acid are well known to those skilled in the art and, for example, may be obtained from a hybridoma producing an antibody against a 2871 receptor polypeptide or fragment thereof. The recombinant DNA encoding the humanized antibody, or fragment thereof, can then be cloned into an appropriate expression vector.

Humanized or CDR-grafted antibodies can be produced by CDR-grafting or CDR substitution, wherein one, two, or all CDR's of an immunoglobulin chain can be replaced. See *e.g.,* U.S. Patent No. 5,225,539; Jones *et al.* (1986) *Nature* 321:552-525; Verhoeyan *et al.* (1988) *Science* 239:1534; Beidler *et al.* (1988) *J*. *Immunol.* 141:4053-4060; Winter US patent No. 5,225,539, the contents of all of which are hereby expressly incorporated by reference. Winter describes a CDR-grafting method which may be used to prepare the humanized antibodies of the present invention (UK Patent Application GB 2188638A, filed on March 26, 1987; Winter US patent No. 5,225,539), the contents of which is expressly incorporated by reference. In one example, a humanized antibody which comprises at least one complementarity determining region (CDR) of the heavy chain (CDR1, CDR2 and/or CDR3) and at least one CDR of the light chain (CDR1, CDR2 and/or CDR3) of a murine monoclonal antibody and a human framework region, can be administered in accordance with the invention.

Antibodies may also be modified by specific deletion of human T cell epitopes or "deimmunized" by the methods disclosed in WO 98/52976 and WO 00/34317, the contents of which are specifically incorporated by reference herein. Briefly, the murine heavy and light chain variable regions of an antibody can be analyzed for peptides which bind to MHC Class II; these peptides represent potential T-cell epitopes (as defined in WO 98/52976 and WO 00/34317). For detection of potential T-cell epitopes, a computer modelling approach termed "peptide threading" can be applied, and in addition a database of human MHC class II binding peptides can be searched for motifs present in the murine V_{H} and V_{L} sequences, as described in WO 98/52976 and WO 00/34317. Potential T-cell epitopes detected can be eliminated by substituting small numbers of amino acid residues in the variable regions. The CDRs and some framework residues important for antibody structure and binding are preferably preserved. As far as possible conservative substitutions are made, often but not exclusively, an amino acid common at this position in human germline antibody sequences may be used. Human germline sequences are disclosed in Tomlinson, I.A. *et al.* (1992) *J. Mol.* *Biol.* 227:776-798; Cook, G. P. *et al.* (1995) *Immunol. Today* Vol. 16 (5): 237-242; Chothia, D. *et al.* (1992) *J*. *Mol. Bio.* 227:799-817. The V BASE directory provides a comprehensive directory of human immunoglobulin variable region sequences (compiled by Tomlinson, I.A. et al. MRC Centre for Protein Engineering, Cambridge, UK). After the deimmunised V_{H} and V_{L} of an antibody are constructed by mutagenesis of the murine V_{H} and V_{L} genes, the mutagenized variable sequence can, optionally, be fused to a human constant region, e.g., human IgG1 or κ constant regions. The recombinant deimmunized antibody can be transfected into a suitable host cell for expression, for example, NS0 or CHO cells, to produce complete recombinant antibodies.

Detection can be facilitated by coupling (i.e., physically linking) an antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

Still further, an antibody (or fragment thereof) can be conjugated to a therapeutic moiety such as a cytotoxin, a therapeutic agent or a radioactive ion. A cytotoxin or cytotoxic agent includes any agent that is detrimental to cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, puromycin, maytansinoids, *e.g.*, maytansinol (see US Patent No. 5,208,020), CC-1065 (see US Patent Nos. 5,475,092, 5,585,499, 5,846,545) and analogs or homologs thereof. Therapeutic agents include, but are not limited to, antimetabolites (*e*.*g*., methotrexate, 6-mercaptopurine, 6-thioguanine, cytarabine, 5-fluorouracil decarbazine), alkylating agents (*e*.*g*., mechlorethamine, thioepa chlorambucil, CC-1065, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines (*e*.*g*., daunorubicin (formerly daunomycin) and doxorubicin), antibiotics (*e*.*g*., dactinomycin (formerly actinomycin), bleomycin, mithramycin, and anthramycin (AMC)), and anti-mitotic agents (*e*.*g*., vincristine, vinblastine, taxol and maytansinoids). Radioactive ions include, but are not limited to iodine, yttrium and praseodymium.

The conjugates of the invention can be used for modifying a given biological response, the therapeutic moiety is not to be construed as limited to classical chemical therapeutic agents. For example, the therapeutic moiety may be a protein or polypeptide possessing a desired biological activity. Such proteins may include, for example, a toxin such as abrin, ricin A, pseudomonas exotoxin, or diphtheria toxin; a protein such as tumor necrosis factor, α-interferon, β-interferon, nerve growth factor, platelet derived growth factor, tissue plasminogen activator; or, biological response modifiers such as, for example, lymphokines, interleukin-1 ("IL-1"), interleukin-2 ("IL-2"), interleukin-6 ("IL-6"), granulocyte macrophase colony stimulating factor ("GM-CSF'), granulocyte colony stimulating factor ("G-CSF"), or other growth factors.

Alternatively, an antibody can be conjugated to a second antibody to form an antibody heteroconjugate as described by Segal in U.S. Patent No. 4,676,980.

To generate antibodies, an isolated receptor polypeptide is used as an immunogen to generate antibodies using standard techniques for polyclonal and monoclonal antibody preparation. Either the full-length protein or antigenic peptide fragment can be used. An antigenic fragment will typically comprise at least 12 contiguous amino acid residues. The antigenic peptide can comprise, however, at least 14 amino acid residues, at least 15 amino acid residues, at least 20 amino acid residues, or at least 30 amino acid residues. In one embodiment, fragments correspond to regions that are located on the surface of the protein, e.g., hydrophilic regions.

An appropriate immunogenic preparation can be derived from native, recombinantly expressed, protein or chemically synthesized peptides.

Anti-2871 receptor antibodies can be used to isolate a receptor protein by standard techniques, such as affinity chromatography or immunoprecipitation. The antibodies can facilitate the purification of the natural receptor protein from cells and recombinantly produced receptor protein expressed in host cells.

The antibodies are useful for inhibiting receptor function, for example, blocking ligand binding. These uses can be applied in a therapeutic context in which treatment involves inhibiting receptor function. An antibody can be used, for example, to block ligand binding. Antibodies can be prepared against specific fragments containing sites required for function or against intact receptor associated with a cell. The invention also encompasses kits for using antibodies to detect the presence of a receptor protein in a biological sample. The kit can comprise antibodies such as a labeled or labelable antibody and a compound or agent for detecting receptor protein in a biological sample; means for determining the amount of receptor protein in the sample; and means for comparing the amount of receptor protein in the sample with a standard. The compound or agent can be packaged in a suitable container. The kit can further comprise instructions for using the kit to detect receptor protein.

### Diagnostic Applications

The 2871 receptor polypeptides also useful to provide a target for diagnosing a disease or predisposition to disease mediated by the receptor protein and/or ligands thereof, such as those disclosed herein, especially asthma, chronic obstructive pulmonary disorder, neutropenia, anemia, and thrombocytopenia. Accordingly, methods are provided for detecting the presence, or levels of, the receptor protein in a cell, tissue, or organism. The method involves contacting a biological sample with a compound capable of interacting with the receptor protein (e.g., an antibody) such that the interaction can be detected.

The receptor protein also provides a target for diagnosing active disease, or predisposition to disease, in a patient having a variant receptor protein. Thus, receptor protein can be isolated from a biological sample, assayed for the presence of a genetic mutation that results in aberrant receptor protein. This includes amino acid substitution, deletion, insertion, rearrangement, (as the result of aberrant splicing events), and inappropriate post-translational modification, which may result in altered interaction with the natural ligand. Analytic methods include altered electrophoretic mobility, altered tryptic peptide digest, altered receptor activity in cell-based or cell-free assay, alteration in ligand or antibody-binding pattern, altered isoelectric point, direct amino acid sequencing, and any other of the known assay techniques useful for detecting mutations in a protein.

*In vitro* techniques for detection of receptor protein include enzyme linked immunosorbent assays (ELISAs), Western blots, immunoprecipitations and immunofluorescence. Alternatively, the protein can be detected *in vivo* in a subject by introducing into the subject a labeled anti-receptor antibody. For example, the antibody can be labeled with a radioactive marker whose presence and location in a subject can be detected by standard imaging techniques. Particularly useful are methods which detect the allelic variant of a receptor protein expressed in a subject and methods which detect fragments of a receptor protein in a sample.

Antibodies directed to 2871 receptor protein are useful to detect the presence of receptor protein in cells or tissues to determine the pattern of expression of the receptor among various tissues in an organism and over the course of normal development.

The antibodies can be used to detect receptor protein *in situ*, *in vitro*, or in a cell lysate or supernatant in order to evaluate the abundance and pattern of expression.

The antibodies can also be used to assess abnormal tissue distribution or abnormal expression or activity during development. Additionally, antibody detection of circulating fragments of the full length receptor protein can be used to identify receptor turnover.

Further, the antibodies can be used to assess receptor expression in disease states such as in active stages of the disease or in a subject with a predisposition toward disease related to receptor function. When a disorder is caused by an inappropriate tissue distribution, developmental expression, or level of expression of the receptor protein, the antibody can be prepared against the normal receptor protein. If a disorder is characterized by a specific mutation in the receptor protein, antibodies specific for this mutant protein can be used to assay for the presence of the specific mutant receptor protein.

The antibodies can also be used to assess normal and aberrant subcellular localization of cells in the various tissues in an organism. Antibodies can be developed against the whole receptor or portions of the receptor, for example, portions of the extracellular domain.

The 2871 receptor polypeptides are also useful in pharmacogenomic analysis. Accordingly, genetic polymorphism may lead to allelic protein variants of the receptor protein in which one or more of the receptor functions in one population is different from those in another population. The polypeptides thus allow a target to ascertain a genetic predisposition that can affect treatment modality. Thus, in a ligand-based treatment, polymorphism may give rise to domains that are more or less active in ligand binding, and receptor activation.
Accordingly, ligand dosage would necessarily be modified to maximize the therapeutic effect within a given population containing a polymorphism. As an alternative to genotyping, specific polymorphic polypeptides could be identified. Thus, antibodies prepared against polymorphic receptor proteins can be used to identify subjects that require modified treatment modalities.

The 2871 receptor polypeptides and modulators are also useful for monitoring therapeutic effects during clinical trials and other treatment. Thus, the therapeutic effectiveness of an agent that is designed to increase or decrease 2871 receptor activity can be monitored over the course of treatment using the receptor polypeptides as an end-point target. Accordingly, where treatment is ultimately aimed at correcting receptor expression level or the presence of aberrant receptors and aberrant tissue distribution or developmental expression, antibodies directed against the receptor or relevant fragments can be used to monitor therapeutic efficacy.

As used herein, the term "treatment", "treating", or "therapy" is defined as the application or administration of an an agent which modulates the binding of a ligand (e.g., AP6A) to 2871 receptor protein, and/or modulates the function of 2871 receptor protein (e.g., including ligands, inhibitors and/or promoters), alone or in combination with, a second agent to a subject, e.g., a patient, or application or administration of the agent to an isolated tissue or cell, e.g., cell line, from a subject, e.g., a patient, who has a disorder (e.g., a disorder as described herein), a symptom of a disorder or a predisposition toward a disorder, with the purpose to cure, heal, alleviate, relieve, alter, remedy, ameliorate, improve or affect the disorder, the symptoms of the disorder or the predisposition toward the disorder. Treating a cell refers to the inhibition, ablation, killing of a cell *in vitro* or *in vivo*, or otherwise reducing capacity of a cell, e.g., an aberrant cell, to mediate a disorder, e.g., a disorder as described herein (e.g., a hematopoietic or proliferative or inflammation disorder). In one embodiment, "treating a cell" refers to a reduction in the activity and/or proliferation of a cell, e.g., a hyperproliferative cell. Such reduction does not necessarily indicate a total elimination of the cell, but a reduction, e.g., a statistically significant reduction, in the activity or the number of the cell.

### Methods of Modulating/Therapy

Modulation of cellular functions (e.g., signaling, chemotaxis, actin polymerization, shape change, respiratory burst, exocytosis, secretion, apoptosis,) induced upon binding of ligand (e.g., an adenosine nucleotide, e.g., AP6A) to 2871 receptor protein provides an effective and selective way of modulating the 2871 receptor mediated functions (e.g., differentiation, proliferation) of cells. Thus, agents which modulate the binding of a ligand (e.g., AP6A) to 2871 receptor protein, including ligands, inhibitors and/or promoters, such as those identified as described herein, can be used to modulate cell function. The 2871 receptor polypeptides are also useful for treating a receptor-associated disorder, such as those described herein. Accordingly, methods for treatment include the use of soluble receptor or fragments of the receptor protein that compete for ligand binding. These receptors or fragments can have a higher affinity for the ligand so as to provide effective competition.

In one aspect, the present invention provides a method of modulating (inhibiting or promoting) a 2871 receptor mediated response in a subject (e.g., a human) in need of such therapy, comprising administering an effective amount of an agent which modulates the binding of adenosine nucleotide (e.g., AP6A) to 2871 receptor protein to a subject in need of such therapy. In one embodiment, an effective amount of an agent which inhibits the binding of an adenosine nucleotide (e.g., AP6A) to 2871 receptor protein is administered to a subject to inhibit inflammation (e.g., to reduce inflammation, to prevent inflammation). For example, antibodies and antigen-binding fragments which bind to 2871 receptor protein and inhibit the binding of adenosine nucleotide (e.g., AP6A) to 2871 receptor protein, or antibodies or antigen-binding fragments thereof which bind an adenosine nucleotide (e.g., AP6A) and inhibit binding of the adenosine nucleotide (e.g., AP6A) to 2871 receptor protein can be used in the method. As a result, one or more processes, such as apoptosis for example, chemotaxis, actin polymerization, shape change, exocytosis (e.g., of enzymes) or mediator release, can be inhibited. According to the method the severity of symptoms associated with a condition (e.g., an inflammatory or hematopoietic condition) can be inhibited (reduced) in whole or in part. Where the subject has a relapsing or chronic condition, an effective amount of an agent which inhibits the binding of an adenosine nucleotide (e.g., AP6A) to 2871 receptor protein can be administered as treatment of a condition, and therapy can be continued (maintenance therapy) with the same or different dosing as indicated, to inhibit relapse or renewed onset of symptoms.

Thus, the invention relates to a method of treating a subject having a 2871 receptor mediated disease, comprising administering to the subject an effective amount of an agent which inhibits the binding of an adenosine nucleotide (e.g., AP6A) to 2871 receptor protein and/or which inhibits the function of 2871 receptor protein upon adenosine nucleotide binding (e.g., AP6A-induced signaling or activation).

In another embodiment, an agent which promotes or augments one or more ligand-induced functions of a mammalian 2871 receptor protein is administered to induce (trigger or enhance) the recruitment of cells to a desired site or to induce a cellular response, such as apoptosis, chemotaxis, actin polymerization, shape change, exocytosis (e.g., of enzymes) or mediator release, resulting in the beneficial stimulation of processes.

In a another embodiment, the invention relates to a method of promoting adenosine nucleotide (e.g., AP6A) induced function of cells (e.g., leukocytes) which express 2871 receptor protein in a subject, comprising administering to the subject an effective amount of an agent which promotes or augments binding of AP6A to 2871 receptor protein, and/or augments one or more adenosine nucleotide (e.g., AP6A) induced functions of a mammalian 2871 receptor protein.

Agents which can inhibit the binding of an adenosine nucleotide (e.g., AP6A) to 2871 receptor protein, including antibodies, such as those described herein, can be used to modulate 2871 receptor protein function. In one aspect, the present invention provides a method of modulating (inhibiting or promoting) a 2871 receptor mediated response in a subject in need of such therapy, comprising administering an effective amount of an agent (e.g., a small organic molecule) which inhibits binding of adenosine nucleotide (e.g., AP6A) to 2871 receptor protein to a subject in need of such therapy. In one embodiment, an effective amount of an agent (e.g., a small organic molecule) which inhibits binding of adenosine nucleotide (e.g., AP6A) to 2871 receptor protein is administered to a subject to inhibit (reduce or prevent) cell activity. As a result, one or more cellular processes, such as apoptosis, chemotaxis, actin polymerization, shape change, exocytosis (e.g., of enzymes) or mediator release, is inhibited. In another embodiment, an effective amount of an agent (e.g., a small organic molecule) which inhibits binding of adenosine nucleotide (e.g., AP6A) to mammalian 2871 receptor protein is administered to a subject to inhibit 2871 receptor ligand-induced function.

The term "subject" is defined herein to includes mammals such as humans, primates, cows, sheep, goats, horses, dogs, cats, rabbits, guinea pigs, rats, mice or other bovine, ovine, equine, canine, feline, rodent or murine species.

Diseases or conditions, including chronic diseases, of humans or other species which can be treated with agents which can inhibit the binding of adenosine nucleotide (e.g., AP6A) to 2871 receptor protein or adenosine nucleotide (e.g., AP6A)-induced functions thereof, as well as cellular activities and function which can be modulated include, but are not limited to those described in the following section.

### Disorders/Cellular Functions

Expression analyses, as well as similarity to other receptors, have implicated roles of the 2871 receptor in the physiology of various disorders including immune, hematologic, neoplastic, and proliferative and/or differentiative disorders (U.S. Patent No. 6,063,596; and PCT Publication Numbers WO 99/63087; WO 01/44474).

Disclosed herein are studies demonstrating 2871 receptor expression in T helper cells (Th cells). The present invention relates to methods and compositions for the modulation, diagnosis, and treatment of 2871 receptor mediated disorders. Particularly relevant are immune and respiratory disorders, especially T helper cell and Th cell-like related disorders.

Immune, *e.g.,* inflammatory, (*e.g.* respiratory inflammatory) disorders or diseases include, but are not limited to, autoimmune diseases (including, for example, diabetes mellitus, arthritis (including rheumatoid arthritis, juvenile rheumatoid arthritis, osteoarthritis, psoriatic arthritis), multiple sclerosis, encephalomyelitis, myasthenia gravis, systemic lupus erythematosis, autoimmune thyroiditis, dermatitis (including atopic dermatitis and eczematous dermatitis), psoriasis, Sjögren's Syndrome, inflammatory bowel disease, *e.g.* Crohn's disease and ulcerative colitis, aphthous ulcer, iritis, conjunctivitis, keratoconjunctivitis, asthma, allergic asthma, chronic obstructive pulmonary disease, cutaneous lupus erythematosus, scleroderma, vaginitis, proctitis, drug eruptions, leprosy reversal reactions, erythema nodosum leprosum, autoimmune uveitis, allergic encephalomyelitis, acute necrotizing hemorrhagic encephalopathy, idiopathic bilateral progressive sensorineural hearing loss, aplastic anemia, pure red cell anemia, idiopathic thrombocytopenia, polychondritis, Wegener's granulomatosis, chronic active hepatitis, Stevens-Johnson syndrome, idiopathic sprue, lichen planus, Graves' disease, sarcoidosis, primary biliary cirrhosis, uveitis posterior, and interstitial lung fibrosis), graft-versus-host disease, cases of transplantation, and allergy such as, atopic allergy.

Respiratory disorders include, but are not limited to, apnea, asthma, particularly bronchial asthma, berillium disease, bronchiectasis, bronchitis, bronchopneumonia, cystic fibrosis, diphtheria, dyspnea, emphysema, chronic obstructive pulmonary disease, allergic bronchopulmonary aspergillosis, pneumonia, acute pulmonary edema, pertussis, pharyngitis, atelectasis, Wegener's granulomatosis, Legionnaires disease, pleurisy, rheumatic fever, and sinusitis.

Disorders involving T-cells include, but are not limited to, cell-mediated hypersensitivity, such as delayed type hypersensitivity and T-cell-mediated cytotoxicity, and transplant rejection; autoimmune diseases, such as systemic lupus erythematosus, Sjögren syndrome, systemic sclerosis, inflammatory myopathies, mixed connective tissue disease, and polyarteritis nodosa and other vasculitides; immunologic deficiency syndromes, including but not limited to, primary immunodeficiencies, such as thymic hypoplasia, severe combined immunodeficiency diseases, and AIDS; leukopenia; reactive (inflammatory) proliferations of white cells, including but not limited to, leukocytosis, acute nonspecific lymphadenitis, and chronic nonspecific lymphadenitis; neoplastic proliferations of white cells, including but not limited to lymphoid neoplasms, such as precursor T-cell neoplasms, such as acute lymphoblastic leukemia/lymphoma, peripheral T-cell and natural killer cell neoplasms that include peripheral T-cell lymphoma, unspecified, adult T-cell leukemia/lymphoma, mycosis fungoides and Sézary syndrome, and Hodgkin disease.

Disorders involving B-cells include, but are not limited to precursor B-cell neoplasms, such as lymphoblastic leukemia/lymphoma. Peripheral B-cell neoplasms include, but are not limited to, chronic lymphocytic leukemia/small lymphocytic lymphoma, follicular lymphoma, diffuse large B-cell lymphoma, Burkitt lymphoma, plasma cell neoplasms, multiple myeloma, and related entities, lymphoplasmacytic lymphoma (Waldenstrõm macroglobulinemia), mantle cell lymphoma, marginal zone lymphoma (MALToma), and hairy cell leukemia.

The 2871 receptor gene is expressed at significant levels in all blood cell progenitors, described in PCT publication nos. WO 99/63087 and WO 01/44474, which are incorporated herein by reference. It is highly expressed in bone marrow (CD34⁺), G-CSF-mobilized peripheral blood (containing circulating progenitors derived from bone marrow) and is moderately expressed in CD34⁺ adult bone marrow and CD34⁺ cord blood cells. It is also highly expressed in megakaryocytes as well as CD41⁺ (CD14⁻) bone marrow cells. G-CSF-mobilized peripheral blood contains circulating progenitors derived from bone marrow. Accordingly, expression of the gene and activity of the receptor protein is relevant for treating hematologic disorders associated with the formation of differentiated and/or mature blood cells. In this regard, disorders that are particularly relevant include anemia, neutropenia, and thrombocytopenia.

In normal bone marrow, the myelocytic series (polymorphonuclear cells) make up approximately 60% of the cellular elements, and the erythrocytic series, 20-30%. Lymphocytes, monocytes, reticular cells, plasma cells and megakaryocytes together constitute 10-20%. Lymphocytes make up 5-15% of normal adult marrow. In the bone marrow, cell types are add mixed so that precursors of red blood cells (erythroblasts), macrophages (monoblasts), platelets (megakaryocytes), polymorphonuclear leucocytes (myeloblasts), and lymphocytes (lymphoblasts) can be visible in one microscopic field. In addition, stem cells exist for the different cell lineages, as well as a precursor stem cell for the committed progenitor cells of the different lineages. The various types of cells and stages of each would be known to the person of ordinary skill in the art and are found, for example, on page 42 (Figure 2-8) of *Immunology, Imunopathology and Immunity*, Fifth Edition, Sell *et al.* Simon and Schuster (1996), incorporated by reference for its teaching of cell types found in the bone marrow. According, the invention is directed to disorders arising from these cells. These disorders include but are not limited to the following: diseases involving hematopoeitic stem cells; committed lymphoid progenitor cells; lymphoid cells including B and T-cells; committed myeloid progenitors, including monocytes, granulocytes, and megakaryocytes; and committed erythroid progenitors. These include but are not limited to the leukemias, including B-lymphoid leukemias, T-lymphoid leukemias, undifferentiated leukemias; erythroleukemia, megakaryoblastic leukemia, monocytic; [leukemias are encompassed with and without differentiation]; chronic and acute lymphoblastic leukemia, chronic and acute lymphocytic leukemia, chronic and acute myelogenous leukemia, lymphoma, myelo dysplastic syndrome, chronic and acute myeloid leukemia, myelomonocytic leukemia; chronic and acute myeloblastic leukemia, chronic and acute myelogenous leukemia, chronic and acute promyelocytic leukemia, chronic and acute myelocytic leukemia, hematologic malignancies of monocyte-macrophage lineage, such as juvenile chronic myelogenous leukemia; secondary AML, antecedent hematological disorder; refractory anemia; aplastic anemia; reactive cutaneous angioendotheliomatosis; fibrosing disorders involving altered expression in dendritic cells, disorders including systemic sclerosis, E-M syndrome, epidemic toxic oil syndrome, eosinophilic fasciitis localized forms of scleroderma, keloid, and fibrosing colonopathy; angiomatoid malignant fibrous histiocytoma; carcinoma, including primary head and neck squamous cell carcinoma; sarcoma, including kaposi's sarcoma; fibroadanoma and phyllodes tumors, including mammary fibroadenoma; stromal tumors; phyllodes tumors, including histiocytoma; erythroblastosis; neurofibromatosis; diseases of the vascular endothelium; demyelinating, particularly in old lesions; gliosis, vasogenic edema, vascular disease, Alzheimer's and Parkinson's disease; T-cell lymphomas; B-cell lymphomas.

Disorders involving precursor T-cell neoplasms include precursor T lymphoblastic leukemia/lymphoma. Disorders involving peripheral T-cell and natural killer cell neoplasms include T-cell chronic lymphocytic leukemia, large granular lymphocytic leukemia, mycosis fungoides and Sézary syndrome, peripheral T-cell lymphoma, unspecified, angioimmunoblastic T-cell lymphoma, angiocentric lymphoma (NK/T-cell lymphoma^{4a}), intestinal T-cell lymphoma, adult T-cell leukemia/lymphoma, and anaplastic large cell lymphoma.

The present invention also relates to methods and compositions for the modulation, diagnosis, and treatment of hematopoeitic disorders involving cells of leukocyte, erythrocyte, and platelet lineages, i.e., the differentiated cells and their less-differentiated progenitors including, but not limited to, erythroblasts, megakaryocytes, and leukocytes that are not fully differentiated, as well as CD34⁺ stem cells. Two identified ligands for 2871, cAMP and cADP-ribose are known to initiate signaling cascades resulting in increased intracellular calcium levels in a number of cell types (Lee (review), 2001, Franco et al, 2001). Increased intracellular calcium levels are known to be important in stimulating proliferation of hematopoietic cells as well as the various blood cell lineages. Signaling initiated by cADP-ribose has been shown to enhance the proliferation of human CD34+ progenitor cells via intracellular calcium release (Podesta et al, 2000). Megakaryocyte proliferation has also been shown to be dependent on intracellular calcium release, and cAMP singalling (Kunitama et al, 1997; Uneyama et al, 1995). Thus, the 2871 receptor-mediated signaling pathway may play a key role in mediating hematopoeitic progenitor cell and megakaryocyte proliferation by controlling intracellular calcium release. Agents targeting this pathway (in particular, agonsits), would be useful for expanding pools of uncommitted hematopoietic progenitors (e.g. in patients with anemia or cytopenias induced by chemo or radiotherapy). Stimulating megakaryopoiesis through the 2871 pathway could be most useful (e.g. in patients with thrombocytopenia induced by chemo/radio therapy during cancer treatment).

This receptor-ligand interaction may also be essential in regulating platelet activation and therefore the methods contained herein useful in the treatment of thrombosis, especially given the high level of expression in platelets and megakaryocytes. Of particular relevance are disorders related to reduced platelet number, thrombocytopenia, including idiopathic thrombocytopenic purpura, acute idiopathic thrombocytopenic purpura, drug-induced thrombocytopenia, HIV-associated thrombocytopenia, and thrombotic microangiopathies: thrombotic thrombocytopenic purpura and hemolytic-uremic syndrome.

Furthermore, the 2871 gene has been shown to be expressed at elevated levels in tumorigenic cells including breast tumor cells, colon tumor cells, lung tumor cells, and ovary tumor cells. See PCT publications WO 99/63087 and WO 01/44474, which are incorporated herein by reference. Additionally, expression of 2871 is downregulated in the presence of p53, further suggesting 2871 may be involved in tumorigenesis, particularly in breast, colon, lung, and ovarian cancer.

As used herein, the terms "cancer," "hyperproliferative" and "neoplastic" refer to cells having the capacity for autonomous growth, i.e., an abnormal state or condition characterized by rapidly proliferating cell growth. Hyperproliferative and neoplastic disease states may be categorized as pathologic, i.e., characterizing or constituting a disease state (e.g., malignancies of the various organ systems, such as affecting breast, lung, and ovary, and adenocarcinomas which include malignancies such as most colon cancers, and non-small cell carcinoma of the lung), or may be categorized as non-pathologic, i.e., a deviation from normal but not associated with a disease state (e.g., proliferation of cells associated with wound repair).

Still further, expression profiles have demonstrated expression of the 2871 protein in placenta, glial cells, brain cortex, aorta, pancreas, prostate, uterus, testis, thymus, tonsils, and skin cells. Such expression profiles further suggest particular roles for 2871 receptor function diverse biological roles in differentiation, and proliferation; 2871 receptor protein may mediate various disorders, including cellular proliferative and/or differentiative disorders of the brain, blood vessels, heart, pancreas, prostate, uterus, testis, and/or thymus.

Disorders involving the brain include, but are not limited to, disorders involving neurons, and disorders involving glia, such as astrocytes, oligodendrocytes, ependymal cells, and microglia; cerebral edema, raised intracranial pressure and herniation, and hydrocephalus; malformations and developmental diseases, such as neural tube defects, forebrain anomalies, posterior fossa anomalies, and syringomyelia and hydromyelia; perinatal brain injury; cerebrovascular diseases, such as those related to hypoxia, ischemia, and infarction, including hypotension, hypoperfusion, and low-flow states--global cerebral ischemia and focal cerebral ischemia--infarction from obstruction of local blood supply, intracranial hemorrhage, including intracerebral (intraparenchymal) hemorrhage, subarachnoid hemorrhage and ruptured berry aneurysms, and vascular malformations, hypertensive cerebrovascular disease, including lacunar infarcts, slit hemorrhages, and hypertensive encephalopathy; infections, such as acute meningitis, including acute pyogenic (bacterial) meningitis and acute aseptic (viral) meningitis, acute focal suppurative infections, including brain abscess, subdural empyema, and extradural abscess, chronic bacterial meningoencephalitis, including tuberculosis and mycobacterioses, neurosyphilis, and neuroborreliosis (Lyme disease), viral meningoencephalitis, including arthropod-borne (Arbo) viral encephalitis, *Herpes simplex* virus Type 1, *Herpes simplex* virus Type 2, *Varicalla-zoster* virus (*Herpes zoster*), cytomegalovirus, poliomyelitis, rabies, and human immunodeficiency virus 1, including HIV-1 meningoencephalitis (subacute encephalitis), vacuolar myelopathy, AIDS-associated myopathy, peripheral neuropathy, and AIDS in children, progressive multifocal leukoencephalopathy, subacute sclerosing panencephalitis, fungal meningoencephalitis, other infectious diseases of the nervous system; transmissible spongiform encephalopathies (prion diseases); demyelinating diseases, including multiple sclerosis, multiple sclerosis variants, acute disseminated encephalomyelitis and acute necrotizing hemorrhagic encephalomyelitis, and other diseases with demyelination; degenerative diseases, such as degenerative diseases affecting the cerebral cortex, including Alzheimer disease and Pick disease, degenerative diseases of basal ganglia and brain stem, including Parkinsonism, idiopathic Parkinson disease (paralysis agitans), progressive supranuclear palsy, corticobasal degenration, multiple system atrophy, including striatonigral degenration, Shy-Drager syndrome, and olivopontocerebellar atrophy, and Huntington disease; spinocerebellar degenerations, including spinocerebellar ataxias, including Friedreich ataxia, and ataxia-telanglectasia, degenerative diseases affecting motor neurons, including amyotrophic lateral sclerosis (motor neuron disease), bulbospinal atrophy (Kennedy syndrome), and spinal muscular atrophy; inborn errors of metabolism, such as leukodystrophies, including Krabbe disease, metachromatic leukodystrophy, adrenoleukodystrophy, Pelizaeus-Merzbacher disease, and Canavan disease, mitochondrial encephalomyopathies, including Leigh disease and other mitochondrial encephalomyopathies; toxic and acquired metabolic diseases, including vitamin deficiencies such as thiamine (vitamin B₁) deficiency and vitamin B₁₂ deficiency, neurologic sequelae of metabolic disturbances, including hypoglycemia, hyperglycemia, and hepatic encephatopathy, toxic disorders, including carbon monoxide, methanol, ethanol, and radiation, including combined methotrexate and radiation-induced injury; tumors, such as gliomas, including astrocytoma, including fibrillary (diffuse) astrocytoma and glioblastoma multiforme, pilocytic astrocytoma, pleomorphic xanthoastrocytoma, and brain stem glioma, oligodendroglioma, and ependymoma and related paraventricular mass lesions, neuronal tumors, poorly differentiated neoplasms, including medulloblastoma, other parenchymal tumors, including primary brain lymphoma, germ cell tumors, and pineal parenchymal tumors, meningiomas, metastatic tumors, paraneoplastic syndromes, peripheral nerve sheath tumors, including schwannoma, neurofibroma, and malignant peripheral nerve sheath tumor (malignant schwannoma), and neurocutaneous syndromes (phakomatoses), including neurofibromotosis, including Type 1 neurofibromatosis (NF1) and TYPE 2 neurofibromatosis (NF2), tuberous sclerosis, and Von Hippel-Lindau disease.

Disorders involving blood vessels include, but are not limited to, responses of vascular cell walls to injury, such as endothelial dysfunction and endothelial activation and intimal thickening; vascular diseases including, but not limited to, congenital anomalies, such as arteriovenous fistula, atherosclerosis, and hypertensive vascular disease, such as hypertension; inflammatory disease--the vasculitides, such as giant cell (temporal) arteritis, Takayasu arteritis, polyarteritis nodosa (classic), Kawasaki syndrome (mucocutaneous lymph node syndrome), microscopic polyanglitis (microscopic polyarteritis, hypersensitivity or leukocytoclastic anglitis), Wegener granulomatosis, thromboanglitis obliterans (Buerger disease), vasculitis associated with other disorders, and infectious arteritis; Raynaud disease; aneurysms and dissection, such as abdominal aortic aneurysms, syphilitic (luetic) aneurysms, and aortic dissection (dissecting hematoma); disorders of veins and lymphatics, such as varicose veins, thrombophlebitis and phlebothrombosis, obstruction of superior vena cava (superior vena cava syndrome), obstruction of inferior vena cava (inferior vena cava syndrome), and lymphangitis and lymphedema; tumors, including benign tumors and tumor-like conditions, such as hemangioma, lymphangioma, glomus tumor (glomangioma), vascular ectasias, and bacillary angiomatosis, and intermediate-grade (borderline low-grade malignant) tumors, such as Kaposi sarcoma and hemangloendothelioma, and malignant tumors, such as angiosarcoma and hemangiopericytoma; and pathology of therapeutic interventions in vascular disease, such as balloon angioplasty and related techniques and vascular replacement, such as coronary artery bypass graft surgery.

Disorders involving the heart, include but are not limited to, heart failure, including but not limited to, cardiac hypertrophy, left-sided heart failure, and right-sided heart failure; ischemic heart disease, including but not limited to angina pectoris, myocardial infarction, chronic ischemic heart disease, and sudden cardiac death; hypertensive heart disease, including but not limited to, systemic (left-sided) hypertensive heart disease and pulmonary (right-sided) hypertensive heart disease; valvular heart disease, including but not limited to, valvular degeneration caused by calcification, such as calcific aortic stenosis, calcification of a congenitally bicuspid aortic valve, and mitral annular calcification, and myxomatous degeneration of the mitral valve (mitral valve prolapse), rheumatic fever and rheumatic heart disease, infective endocarditis, and noninfected vegetations, such as nonbacterial thrombotic endocarditis and endocarditis of systemic lupus erythematosus (Libman-Sacks disease), carcinoid heart disease, and complications of artificial valves; myocardial disease, including but not limited to dilated cardiomyopathy, hypertrophic cardiomyopathy, restrictive cardiomyopathy, and myocarditis; pericardial disease, including but not limited to, pericardial effusion and hemopericardium and pericarditis, including acute pericarditis and healed pericarditis, and rheumatoid heart disease; neoplastic heart disease, including but not limited to, primary cardiac tumors, such as myxoma, lipoma, papillary fibroelastoma, rhabdomyoma, and sarcoma, and cardiac effects of noncardiac neoplasms; congenital heart disease, including but not limited to, left-to-right shunts--late cyanosis, such as atrial septal defect, ventricular septal defect, patent ductus arteriosus, and atrioventricular septal defect, right-to-left shunts--early cyanosis, such as tetralogy of fallot, transposition of great arteries, truncus arteriosus, tricuspid atresia, and total anomalous pulmonary venous connection, obstructive congenital anomalies, such as coarctation of aorta, pulmonary stenosis and atresia, and aortic stenosis and atresia, and disorders involving cardiac transplantation.

Disorders involving the pancreas include those of the exocrine pancreas such as congenital anomalies, including but not limited to, ectopic pancreas; pancreatitis, including but not limited to, acute pancreatitis; cysts, including but not limited to, pseudocysts; tumors, including but not limited to, cystic tumors and carcinoma of the pancreas; and disorders of the endocrine pancreas such as, diabetes mellitus; islet cell tumors, including but not limited to, insulinomas, gastrinomas, and other rare islet cell tumors.

Disorders involving the prostate include, but are not limited to, inflammations, benign enlargement, for example, nodular hyperplasia (benign prostatic hypertrophy or hyperplasia), and tumors such as carcinoma.

Disorders involving the uterus include, but are not limited to, benign prostatic hypertrophy or myoma of the uterus, endometriosis, polycystic ovarian disease, and uterine fibroids.

Disorders involving the testis and epididymis include, but are not limited to, congenital anomalies such as cryptorchidism, regressive changes such as atrophy, inflammations such as nonspecific epididymitis and orchitis, granulomatous (autoimmune) orchitis, testicular tumors including germ cell tumors that include, but are not limited to, seminoma, spermatocytic seminoma, embryonal carcinoma, yolk sac tumor choriocarcinoma, teratoma, and mixed tumors, tumore of sex cord-gonadal stroma including, but not limited to, leydig (interstitial) cell tumors and sertoli cell tumors (androblastoma), and testicular lymphoma, and miscellaneous lesions of tunica vaginalis.

Disorders involving the thymus include developmental disorders, such as DiGeorge syndrome with thymic hypoplasia or aplasia; thymic cysts; thymic hypoplasia, which involves the appearance of lymphoid follicles within the thymus, creating thymic follicular hyperplasia; and thymomas, including germ cell tumors, lynphomas, Hodgkin disease, and carcinoids. Thymomas can include benign or encapsulated thymoma, and malignant thymoma Type I (invasive thymoma) or Type II, designated thymic carcinoma.

### Modes of Administration

According to the method, one or more agents can be administered to the subject by an appropriate route, either alone or in combination with another drug. An effective amount of an agent (e.g., an antibody or small organic molecule which binds 2871 receptor and inhibits binding of AP6A to 2871 receptor) is administered. An effective amount is an amount sufficient to achieve the desired therapeutic or prophylactic effect, under the conditions of administration, such as an amount sufficient to inhibit binding of AP6A to 2871 receptor, and thereby, inhibit an inflammatory response. The agents can be administered in a single dose or multiple doses. The dosage can be determined by methods known in the art and is dependent, for example, upon the agent chosen, the subject's age, sensitivity and tolerance to drugs, and overall well-being. Typically, an effective amount can range from about 0.01 mg per day to about 100 mg per day for an adult. Preferably, the dosage ranges from about 1 mg per day to about 100 mg per day or from about 1 mg per day to about 10 mg per day. Antibodies and antigen-binding fragments thereof such as human, humanized, deimmunized, and chimeric antibodies and antigen-binding fragments can often be administered with less frequency than other types of therapeutics. For example, an effective amount of an antibody can range from about 0.01 mg/kg to about 5 or 10 mg/kg administered daily, weekly, biweekly or monthly.

A variety of routes of administration are possible including, for example, oral, dietary, topical, transdermal, rectal, parenteral (e.g., intravenous, intraarterial, intramuscular, subcutaneous, intradermal injection), and inhalation (e.g., intrabronchial, intranasal or oral inhalation, intranasal drops) routes of administration, depending on the agent and disease or condition to be treated. Administration can be local or systemic as indicated. The preferred mode of administration can vary depending upon the agent chosen (e.g., small organic molecule or antibody), and the condition (e.g., disease) being treated, however, oral or parenteral administration is generally preferred.

The agent can be administered as a neutral compound or as a salt. Salts of compounds containing an amine or other basic group can be obtained, for example, by reacting with a suitable organic or inorganic acid, such as hydrogen chloride, hydrogen bromide, acetic acid, perchloric acid and the like. Compounds with a quaternary ammonium group also contain a counteranion such as chloride, bromide, iodide, acetate, perchlorate and the like. Salts of compounds containing a carboxylic acid or other acidic functional group can be prepared by reacting with a suitable base, for example, a hydroxide base. Salts of acidic functional groups contain a countercation such as sodium, potassium and the like.

The agent can be administered to the subject as part of a pharmaceutical composition for modulation (e.g., inhibition) of ligand-induced 2871 receptor function comprising an inhibitor or promotor of ligand-induced 2871 receptor function and a pharmaceutically or physiologically acceptable carrier. Formulation will vary according to the route of administration selected (e.g., solution, emulsion, capsule). Suitable pharmaceutical and physiological carriers can contain inert ingredients which do not interact with the agent (e.g, inhibitor of AP6A binding to 2871 receptor). Standard pharmaceutical formulation techniques can be employed, such as those described in Remington's Pharmaceutical Sciences, Mack Publishing Company, Easton, PA. Suitable pharmaceutical carriers for parenteral administration include, for example, sterile water, physiological saline, bacteriostatic saline (saline containing about 0.9% mg/ml benzyl alcohol), phosphate-buffered saline, Hank's solution, Ringer's-lactate and the like. Methods for encapsulating compositions (such as in a coating of hard gelatin or cyclodextran) are known in the art (Baker, *et al*., "Controlled Release of Biological Active Agents", John Wiley and Sons, 1986). For inhalation, the agent can be solubilized and loaded into a suitable dispenser for administration (e.g., an atomizer, nebulizer or pressurized aerosol dispenser).

Furthermore, where the agent is a protein or peptide, the agent can be administered via *in vivo* expression of the recombinant protein. *In vivo* expression can be accomplished via somatic cell expression according to suitable methods (see, e.g. U.S. Patent No. 5,399,346). In this embodiment, a nucleic acid encoding the protein can be incorporated into a retroviral, adenoviral or other suitable vector (preferably, a replication deficient infectious vector) for delivery, or can be introduced into a transfected or transformed host cell capable of expressing the protein for delivery. In the latter embodiment, the cells can be implanted (alone or in a barrier device), injected or otherwise introduced in an amount effective to express the protein in a therapeutically effective amount.

The present invention will now be illustrated by the following Examples, which are not intended to be limiting in any way.

### EXAMPLES

### Example 1: Screening Assay Methods

### Mammalian Cell Expression

The 2871 receptor proteins were expressed in either human embryonic kidney 293 (HEK293) cells or CHO cells, both of which were stably transfected with cDNA encoding the promiscuous G-protein Gal6. A pEF6 vector (invitrogene) including a cDNA of 2871 comprising only the open reading frame (ORF) or 5'-myc tagged ORF was constructed. Cells were transfected with individual construct by lipofectamine (GIBCO) and selected in the presence of 20 ug/ml Blasticidine. After 3 weeks of selection, drug resistant cells were subcloned to obtain individual clones by limited dilution. To isolate cell lines stably expressing the 2871 receptor protein, about 48 clones were typically selected and analyzed. The expression level for myc-tagged 2871 on individual clones was evaluated by using anti-myc Ab staining and FACScan analysis. The expression level for un-tagged 2871 was evaluated by mRNA analysis using standard Taqman techniques and comparing with mRNA expression of myc-tagged 2871 clones. HEK293G16 or CHOG16 cells transfected with the vector alone serve as negative controls. The clones with the highest expression of 2871 as well as good morphology and growth were used for the screening experiments.

### Functional Screening Assays

GPCRs have been shown to be coupled to activation of phospholipase-C and calcium mobilization. Mammalian cells expressing recombinant GPCRs have been shown to demonstrate similar functional responses. The functional screening procedure involves the use of transfected mammalian cells (CHO, HEK293, RBL, L1.2 etc.) containing a cDNA encoding the 2871 peptides to express the receptor on the cell surface. The cells were loaded with a calcium dependent dye such as fura 2 that produces a fluorescent signal when bound to calcium.

Cells were bound to a receptor agonist, such as Ap6A. Any change in fluorescent signal was measured over a defined period of time using a Fluorescence Imaging Plate Reader (FLIPR) (See Sullivan E, et al. Measurement of [Ca²⁺] using the Fluorometric Imaging Plate Reader (FLIPR). Methods Mol Biol 1999;114:125-133.)

A change in the fluorescence signal pattern generated by the ligand indicates that a compound is a potential agonist for the receptor. Cells were exposed to putative ligands and/or modulators and evaluated for agonist induced calcium mobilization. Cell lines transfected with vector alone served as negative controls in the same screening procedures for agents initially identified as putative ligands and/or modulators.

In addition to calcium mobilization, GPCRs have been shown to be coupled to cAMP stimulation or inhibition. As an alternative methodology to the above described assays, standard cAMP quantitation assays can be substituted for monitoring of calcium flux, as they are also amenable to use in high throughput screening.

### Ligand Binding Assays

Binding assays provide a direct method for ascertaining receptor pharmacology and are adaptable to a high throughput format. Purified ligand for 2871 receptor is labeled, preferably radiolabeled to high specific activity (50-2000 Ci/mmol), for binding studies. A determination is then made that the process of labeling does not diminish the binding activity of the ligand for the 2871 receptor. Assay conditions for buffers, ions, pH and other modulators such as nucleotides may be optimized to establish preferable signal to noise ratio for both membrane and whole cell 2871 receptor sources. For binding assays, specific receptor binding is defined as total associated radioactivity minus the radioactivity measured in the presence of an excess of unlabeled competing ligand (identical to that of the labeled version). Where possible, more than one competing ligand is used to define residual nonspecific binding.

### Microphysiometric Assays

Activation of a wide variety of secondary messenger systems results in extrusion of small amounts of acid from a cell. The acid formed is largely as a result of the increased metabolic activity required to fuel the intracellular signaling process. pH changes in the media surrounding the cell are very small but are detectable by a CYTOSENSOR microphysiometer (Molecular Devices Ltd., Menlo Park, Calif.). Activation of the intracellular signaling pathway effected through 2871 agonism is thus detectable using the CYTOSENSOR.

### Example 2: 2871 Receptor Ligand Identification

A library of putative natural agonists for 2871 was assembled from commercial sources, and screened in this assay. The library incorporated bio-active lipids and nucleotide compounds, with approximately 210 bio-active lipids (Biomol) and approximately 62 nucleotides (Sigma).

2871 receptor- transfectants (HEK293 (60,000 cells/well) or CHO cells (30,000 cells/well)) were seeded into poly-D-lysine-coated 96-well black-wall, clear-bottom microtiter plates 24 hours prior to assay. Cells were loaded for 1 hour with 2 µM Fluo-3-AM fluorescent indicator dye (Molecular Probes) in assay buffer (Hank's balanced salt solution, 10 mM HEPES, 0.1% BSA, 2.5 mM probenecid), washed three times with assay buffer, and then applied to FLIPR for analysis.

Bio-active lipids were screened at final concentration of 200 nM and 1 µM. Nucleotides were screened at final concentration of 1 µM and 2 µM. The ratio of net change (maximum subtracting minimum) in fluorescence over baseline was used to determine agonist response. Positive responses were confirmed by checking original raw responsive curve.

No significant 2871-dependent positive response was detected with bio-active lipids tested for 2871 transfected cell lines comparing with the negative control cell lines. Several 2871-dependent positive responses were identified from the nucleotide library (see Table 2).

**TABLE 2**

| **Agonist activity of Nucleotides on 2871 K293G16** | |
|---|---|
| **Agonist activity** | **No Agonist activity** |
| AMP | ADP |
| AP6A | ADP-glucose |
| AP5A | cAMP |
| AP4A | dATP |
| AP3A | 5'S-ATP |
| AP2A | 8-Bromo-cyclic ADP-robose |
| ADP-mannose | 3-Aminopyridine ADP |
| ADP-ribose | And other type NT |

A dose-dependent (EC50) calcium mobilization response was detected following addition of AMP, AP6A, AP5A, AP4A, AP3A, AP2A, ADP-mannose, and ADP-ribose. Concentration response curve data were fitted by Graphpad Prism 2.1 (Graphpad software, Inc., San Diego). Agonist potency to 2871 is AP6A>AMP≥ADP-ribose>AP5A≥AP4A in terms of EC50. Table 3 depicts results of calcium mobilization analyses of HEK293G16 cells stably transfected with 2871 receptor protein. Results obtained demonstrate calcium mobilization in reponse to adenosine nucleotides in a dose-dependent manner.

**TABLE 3**

| **Agonist Potency for 2871 Ligands** | |
|---|---|
| Compounds by FLIPR assay | EC₅₀ |
| AP₆A | 200 - 400 nM |
| AMP | 500 -800 nM |
| ADP-ribose | 400 - 800 nM |
| AP₅A | 1200 - 1800 |
| nM | |
| AP₄A | 1800 - 2000 |
| nM | |

Furthermore, calcium mobilization response was selectively desensitized treatment with adenosine nucleotide was followed by subsequent treatment with additional adenosine nucleotide. Initial treatment with adenosine nucleotide was followed by additional treatment five to six minutes after initial dose. Treatment and data analysis were as described *supra*. Ligand AP6A effectively desensitized 2871 receptor to subsequent treatment with either AP6A or AMP; while initial AMP treatment conferred desensitization to subsequent AMP treatment, and AP6A retained agonist activity.

### Example 3: Gene Expression in T-Helper Cells by TaqMan Quantitative PCR

Total RNA from various tissues was extracted using a single step method according to the manufacturer instructions (RNA STAT-60 TelTest, Inc). Each RNA preparation was treated with DNase I (Ambion) at 37°C for 1 hour. After phenol extraction the sample was subjected to reverse transcription using the Superscript kit according to the manufacturer instructions (GibcoBRL). A negative control sample which contains RNA but without reverse transcriptase was carried out simultaneously. Mock reverse transcribed samples generated in the absence of reverse transcriptase were run for each RNA/cDNA sample to make sure the DNase I treatment was complete. The integrity of the RNA samples following DNase I treatment was checked by agarose gel electrophoresis and ethidium bromide staining. Samples are determined to have complete DNase I treatment if at least 38 amplification cycles are required to reach threshold levels of flourescence using the internal reference amplicon β-2 microglobulin.

Probes were designed by PrimerExpress software from PE Biosystems using consensus sequence. The primer and probe sequences for RNA expression analysis of gene 2871 is as following:

2871 forward primer 5'ATCGTGTTCCTTGGGCTGAT3' (SEQ ID NO: 12)
2871 reverse primer 5'TCCGAGAGTCCCCAAATGG3' (SEQ ID NO: 13)
2871 probe 5'AGCATTGATCGCTATCTGAAGGTGGTCAA3' (SEQ ID NO:
14)

The target probe gene 2871 was labeled using 6-carboxyfluorescein (FAM). The internal reference amplicon for β2-microglobulin was labeled using VIC. In this way levels of the target gene and internal reference gene can be measured in the same tube by multiplex PCR. Forward and reverse primers and the probes for both the internal reference gene and target gene are added to the TaqMan Universal PCR Master Mix (PE Applied Biosystems). Although final concentrations of primer and probe may vary, they are internally consistent within a given experiment. A typical experiment contains 200nM forward and reverse primers plus 100nM probe for β-2 microglobulin and 600nM forward and reverse primers plus 200nM probe for the target gene. TaqMan matrix experiments are carried out on ABI PRPSM 7700 Sequence Detection System (PE Applied Biosystems). The thermal cycler condition is as follows: hold 2 min at 50°C and 10 min at 95°C, followed by two step PCR for 40 cycles, melt at 95°C for 15 sec and anneal/extend at 60°C 1 min.

RNA from *in vitro* differentiated helper T cell populations that were stimulated with antibodies to CD3 and antibodies to CD28 (αCD3/αCD28 stimulation) for 0 to 24 hours was analyzed. Tho, Th1 and Th2 effector cells were generated from common antigen-specific Thp cells under the influence of IL-12 + anti-IL-4 mAbs and IL-4 + anti-IL-12 mAb. Briefly, purified naive CD4+ Th cells were isolated from peripheral blood, Th0 lines, producing both IFNg and IL-4 were produced by αCD3/αCD28 stimulation in the presence of rIL-2. To generate IL-4 single producing Th2 lines, IL-4 (500 U/ml) is added; to generate IFNg single producing Th1 lines, IL-12 (100 U/ml) is added during this primary stimulation. To propagate the Th0 lines, nothing is added; to propagate the Th2 lines, IL-4 is added plus now also anti-IL-12, because the feeder cells will produce some IL-12; to propagate the Th1 cells, IL-12 is added plus anti-IL-4. Finally, cells were subjected to αCD3/αCD28 stimulation for 0 to 24 hours was analyzed.

A comparative Ct method was used for relative quantitation of gene expression. The threshold cycle or Ct value was the cycle at which a statistically significant increase in ΔRn is detected. A lower Ct value indicates a higher concentration of the mRNA for the gene corresponding to the target probe sequence. The Ct value for the target gene was normalized relative to the internal reference gene Ct value to generate a delta Ct value using the following formula: ΔCt=Ct _{target} - Ct _{reference}. To generate values for relative expression, a cDNA sample with a relatively low expression level in the matrix was chosen as a calibrator sample. The ΔCt value for the calibrator tissue was then subtracted from the ΔCt for each according to the following formula: ΔΔCt=ΔCt-ₛₐₘₚₗₑ - ΔCt-_{calibrator}. A value used for relative expression is calculated using the arithmetic formula given by 2 ^{-ΔΔCt}. This value was then used to graph the relative expression of a the target gene in the multiple tissues in the study.

**Table 4:**

| **2871 Expression** | |
|---|---|
| Cell Type | Relative Expression |
| THO(RL)-0 | 0.04 |
| THO(RL)-1 | 0.23 |
| THO(RL)-6 | 6.62 |
| THO(RL)-24 | 1.56 |
| TH1(RL)-0 | 0.01 |
| TH1(RL)-1 | 0.07 |
| TH1(RL)-6 | 0.48 |
| TH1(RL)-24 | 0.23 |
| TH2(RL)-0 | 0.01 |
| TH2(RL)-1 | 0.16 |
| TH2(RL)-6 | 7.81 |
| TH2(RL)-24 NTC | 1.24 |

All publications including, but not limited to, patents and patent applications, cited in this specification, are herein incorporated by reference.

Although the invention has been described in connection with specific preferred embodiments, it should be understood that the invention as claimed should not be unduly limited to such specific embodiments. While specific terms are employed, they are used as in the art unless otherwise indicated. Various modifications and variations of the described methods and systems of the invention will be apparent to those skilled in the art without departing from the scope and spirit of the invention. Indeed, modifications of the described modes for carrying out the invention which are obvious to those skilled in molecular biology or related fields are intended to be within the scope of the following claims.

## Claims

1. A method of detecting or identifying an agent that binds a 2871 receptor protein or a ligand binding fragment thereof comprising:
a) combining an agent selected from the group consisting of bioactive lipids, proteolipids and nucleotides, to be tested with a composition comprising 2871 receptor protein or a ligand binding fragment thereof under conditions suitable for binding of a ligand to the 2871 receptor protein; and
b) detecting or measuring the formation of a complex between the agent and the 2871 receptor protein or a ligand binding fragment thereof, to thereby identify an agent that binds to the 2871 receptor.

2. A method of identifying an agent that modulates binding of an adenosine nucleotide or analog thereof to a 2871 receptor protein, or adenosine nucleotide binding fragment thereof, comprising:
a) combining an agent to be tested and an adenosine nucleotide or analog thereof with a composition comprising the 2871 receptor protein or an adenosine nucleotide binding fragment thereof, under conditions suitable for binding of the adenosine nucleotide or analog thereof to the receptor protein; and
b) detecting or measuring the formation of a complex between the receptor protein and the adenosine nucleotide or analog thereof, whereby a change in the amount of complex formation by the agent is indicative that the agent modulates binding of the adenosine nucleotide or analog thereof to the receptor protein, or
a) combining an agent to be tested and an adenosine nucleotide or analog thereof with a composition comprising a 2871 receptor fusion protein or an adenosine nucleotide binding fragment thereof, under conditions suitable for binding of the adenosine nucleotide or analog thereof to the 2871 receptor protein; and
b) detecting or measuring the formation of a complex between the receptor protein and the adenosine nucleotide or analog thereof, whereby a change in the amount of complex formation by the compound is indicative that the compound modulates binding of the adenosine nucleotide or analog thereof to the 2871 receptor protein.

3. The method of claim 1 or claim 2, wherein the receptor protein or the agent to be tested comprises a detectable label.

4. The method of claim 3, wherein the label is selected from the group consisting of a radioisotope, an epitope tag, an affinity label, an enzyme, a fluorescent group, a chemiluminescent group, and a chromoluminescent group.

5. The method of claim 1 or claim 2, wherein the composition comprising the 2871 receptor protein or a ligand binding fragment thereof is a cell that expresses any of a 2871 receptor protein, a fusion protein comprising a 2871 receptor protein or a ligand binding fragment of the 2871 receptor protein.

6. The method of claim 1 or claim 2, wherein the composition comprising the 2871 receptor protein or a ligand binding fragment thereof is a membrane preparation comprising the 2871 receptor protein, a fusion protein comprising a 2871 receptor protein or a ligand binding fragment of the 2871 protein.

7. The method of claim 1, wherein the method is a competition assay, in which binding is determined in the presence of at least one ligand that binds the 2871 receptor protein.

8. The method of claim 7, wherein the ligand is an adenosine nucleotide, or analog thereof.

9. The method of claim 8, wherein the ligand is selected from the group consisting of AP6A, AMP, AP5A, AP4A and ADP-ribose.

10. The method of claim 1 or claim 2, wherein the 2871 receptor protein or ligand binding fragment thereof can mediate cellular signaling or a cellular response, and the formation of a complex is monitored by detecting a signaling activity or cellular response of the 2871 receptor protein in response thereto.

11. The method of claim 10 wherein the signaling activity or cellular response is selected from the group consisting of Ca²⁺ flux, pH flux, cAMP flux, chemotaxis, actin polymerization, shape change, respiratory burst, differentiation, proliferation, mediator release and apoptosis.

12. The method of claim 2, wherein the adenosine nucleotide is selected from the group consisting of AP6A, AMP, AP5A, AP4A and ADP-ribose.

13. The method of claim 2, wherein the agent to be tested is an antibody or antibody fragment.

14. A method of identifying a modulator of a 2871 receptor protein comprising:
a) combining an agent to be tested with a cell expressing a 2871 receptor protein or ligand binding fragment thereof under conditions suitable for detecting a 2871 receptor activity; and
b) assessing the ability of the agent to modulate the 2871 receptor activity, whereby modulation of the 2871 receptor activity by the agent is indicative that the agent is a modulator of the 2871 receptor protein; or
a) combining an agent to be tested with a cell expressing a 2871 receptor fusion protein or ligand binding fragment thereof under conditions suitable for detecting a 2871 receptor activity; and
b) assessing the ability of the agent to modulate the 2871 receptor activity, whereby modulation of the 2871 receptor activity by the agent is indicative that the agent is a modulator of the 2871 receptor protein.

15. The method of claim 14, wherein the 2871 receptor activity is a signaling activity or a cellular response.

16. The method of claim 15 wherein the signaling activity or cellular response is selected from the group consisting of Ca²⁺ flux, pH flux, cAMP flux, chemotaxis, actin polymerization, shape change, respiratory burst, differentiation, proliferation, mediator release and apoptosis.

17. A modulator of a 2871 receptor protein identified according to the method of claim 1 or claim 2, wherein the modulator is an inhibitor, or a promoter.

18. The modulator of claim 17 wherein the inhibitor is an antagonist.

19. The modulator of claim 17, wherein the promoter is an agonist.

20. A method of modulating an activity of cells that express a 2871 receptor protein comprising:
contacting the cells with an adenosine nucleotide or analog thereof under conditions sufficient for modulation, thereby modulating activity of the cells;
contacting the cells with an agent that modulates binding of an adenosine nucleotide to 2871 receptor protein under conditions sufficient for modulation, thereby modulating activity of the cells;
and/or contacting the cells with an agent that modulates 2871 receptor protein activity under conditions sufficient for modulation, thereby modulating activity of the cells.

21. The method of claim 20 wherein the adenosine nucleotide is selected from the group consisting of AP6A, AMP, AP5A, AP4A and ADP-ribose.

22. The method of claim 20 wherein the cells are selected from the group consisting of hematopoeitic cells, glial cells, epithelial cells, pancreatic cells, and cardiac cells; or breast tumor cells, ovary tumor cells, lung tumor cells, and colon tumor cells..

23. The method of claim 22 wherein hematopoeitic cells are selected from the group consisting of red blood cells, macrophages, platelets, polymorphonuclear leucocytes, and lymphocytes.

24. The method of claim 22 wherein the lung tumor cells are NCI-H125 lung adenosquamous carcinoma cells.

25. The method of claim 20 wherein the activity of cells which is modulated is selected from the group consisting of Ca²⁺ flux, pH flux, cAMP flux, chemotaxis, actin polymerization, shape change, respiratory burst, differentiation, proliferation, mediator release and apoptosis.

26. A kit comprising reagents used for the methods of any of claims 1, 2, or 14, wherein the reagents comprise an adenosine nucleotide or analog thereof that specifically binds to the 2871 receptor protein, or ligand binding fragment thereof.

27. The kit of claim 26 further comprising means for determining the amount of receptor protein complex in the sample and means for comparing the amount of receptor protein complex in the sample with a standard, and/or instructions and a suitable container.


SEQUENCE LISTING

<110> Millennium Pharmaceuticals, Inc.

<120> METHODS FOR IDENTIFICATION OF LIGANDS OR
      MODULATORS OF 2871 RECEPTOR, A G-PROTEIN COUPLED RECEPTOR

<130> 347-4

<150> US SN 10/010,546
<151> 2001-10-26

<160> 14

<170> FastSEQ for Windows Version 4.0

<210> 1
<211> 358
<212> PRT
<213> Homo sapiens

<400> 1
Met Gly Phe Asn Leu Thr Leu Ala Lys Leu Pro Asn Asn Glu Leu His
 1               5                  10                  15
Gly Gln Glu Ser His Asn Ser Gly Asn Arg Ser Asp Gly Pro Gly Lys
            20                  25                  30
Asn Thr Thr Leu His Asn Glu Phe Asp Thr Ile Val Leu Pro Val Leu
        35                  40                  45
Tyr Leu Ile Ile Phe Val Ala Ser Ile Leu Leu Asn Gly Leu Ala Val
    50                  55                  60
Trp Ile Phe Phe His Ile Arg Asn Lys Thr Ser Phe Ile Phe Tyr Leu
65                  70                  75                  80
Lys Asn Ile Val Val Ala Asp Leu Ile Met Thr Leu Thr Phe Pro Phe
                85                  90                  95
Arg Ile Val His Asp Ala Gly Phe Gly Pro Trp Tyr Phe Lys Phe Ile
            100                 105                 110
Leu Cys Arg Tyr Thr Ser Val Leu Phe Tyr Ala Asn Met Tyr Thr Ser
        115                 120                 125
Ile Val Phe Leu Gly Leu Ile Ser Ile Asp Arg Tyr Leu Lys Val Val
    130                 135                 140
Lys Pro Phe Gly Asp Ser Arg Met Tyr Ser Ile Thr Phe Thr Lys Val
145                 150                 155                 160
Leu Ser Val Cys Val Trp Val Ile Met Ala Val Leu Ser Leu Pro Asn
                165                 170                 175
Ile Ile Leu Thr Asn Gly Gln Pro Thr Glu Asp Asn Ile His Asp Cys
            180                 185                 190
Ser Lys Leu Lys Ser Pro Leu Gly Val Lys Trp His Thr Ala Val Thr
        195                 200                 205
Tyr Val Asn Ser Cys Leu Phe Val Ala Val Leu Val Ile Leu Ile Gly
    210                 215                 220
Cys Tyr Ile Ala Ile Ser Arg Tyr Ile His Lys Ser Ser Arg Gln Phe
225                 230                 235                 240
Ile Ser Gln Ser Ser Arg Lys Arg Lys His Asn Gln Ser Ile Arg Val

                    245                     250                     255
Val Val Ala Val Phe Phe Thr Cys Phe Leu Pro Tyr His Leu Cys Arg
            260                     265                     270
Ile Pro Phe Thr Phe Ser His Leu Asp Arg Leu Leu Asp Glu Ser Ala
        275                     280                     285
Gln Lys Ile Leu Tyr Tyr Cys Lys Glu Ile Thr Leu Phe Leu Ser Ala
    290                     295                     300
Cys Asn Val Cys Leu Asp Pro Ile Ile Tyr Phe Phe Met Cys Arg Ser
305                     310                     315                     320
Phe Ser Arg Arg Leu Phe Lys Lys Ser Asn Ile Arg Thr Arg Ser Glu
                    325                     330                     335
Ser Ile Arg Ser Leu Gln Ser Val Arg Arg Ser Glu Val Arg Ile Tyr
            340                     345                     350
Tyr Asp Tyr Thr Asp Val
        355

<210> 2
<211> 1489
<212> DNA
<213> Homo sapiens

<220>
<221> CDS
<222> (329)...(1405)

<400> 2
   ccacgcgtcc ggagaatttg aaagggtgcc ccaaaggaca atctctaaag gggtaaggga
   60
   gatacctacc ttgtctggta ggggagatgt ttcgttttca tgctttacca gaaaatccac
   120
   ttccctgccg accttagttt caaagcttat tcttaattag agacaagaaa cctgtttcaa
   180
   cttgaagaca ccgtatgagg tgaatggaca gccagccacc acaatgaaag aaatcaaacc
   240
   aggaataacc tatgctgaac ccacgcctca atcgtcccca agtgtttcct gacacgcatc
   300
   tttgcttaca gtgcatcaca actgaaga atg ggg ttc aac ttg acg ctt gca
   352
                                 Met Gly Phe Asn Leu Thr Leu Ala
                                  1               5
   aaa tta cca aat aac gag ctg cac ggc caa gag agt cac aat tca ggc
   400
   Lys Leu Pro Asn Asn Glu Leu His Gly Gln Glu Ser His Asn Ser Gly
        10                  15                  20
   aac agg agc gac ggg cca gga aag aac acc acc ctt cac aat gaa ttt
   448
   Asn Arg Ser Asp Gly Pro Gly Lys Asn Thr Thr Leu His Asn Glu Phe
    25                  30                  35                  40
   gac aca att gtc ttg ccg gtg ctt tat ctc att ata ttt gtg gca agc
   496
   Asp Thr Ile Val Leu Pro Val Leu Tyr Leu Ile Ile Phe Val Ala Ser
   45 50 55
   atc ttg ctg aat ggt tta gca gtg tgg atc ttc ttc cac att agg aat
   544
   Ile Leu Leu Asn Gly Leu Ala Val Trp Ile Phe Phe His Ile Arg Asn
   60 65 70
   aaa acc agc ttc ata ttc tat ctc aaa aac ata gtg gtt gca gac ctc
   592
   Lys Thr Ser Phe Ile Phe Tyr Leu Lys Asn Ile Val Val Ala Asp Leu
   75 80 85
   ata atg acg ctg aca ttt cca ttt cga ata gtc cat gat gca gga ttt
   640
   Ile Met Thr Leu Thr Phe Pro Phe Arg Ile Val His Asp Ala Gly Phe
   90 95 100
   gga cct tgg tac ttc aag ttt att ctc tgc aga tac act tca gtt ttg
   688
   Gly Pro Trp Tyr Phe Lys Phe Ile Leu Cys Arg Tyr Thr Ser Val Leu
   105 110 115 120
   ttt tat gca aac atg tat act tcc atc gtg ttc ctt ggg ctg ata agc
   736
   Phe Tyr Ala Asn Met Tyr Thr Ser Ile Val Phe Leu Gly Leu Ile Ser
   125 130 135
   att gat cgc tat ctg aag gtg gtc aag cca ttt ggg gac tct cgg atg
   784
   Ile Asp Arg Tyr Leu Lys Val Val Lys Pro Phe Gly Asp Ser Arg Met
   140 145 150
   tac agc ata acc ttc acg aag gtt tta tct gtt tgt gtt tgg gtg atc
   832
   Tyr Ser Ile Thr Phe Thr Lys Val Leu Ser Val Cys Val Trp Val Ile
   155 160 165
   atg gct gtt ttg tct ttg cca aac atc atc ctg aca aat ggt cag cca
   880
   Met Ala Val Leu Ser Leu Pro Asn Ile Ile Leu Thr Asn Gly Gln Pro
   170 175 180
   aca gag gac aat atc cat gac tgc tca aaa ctt aaa agt cct ttg ggg
   928
   Thr Glu Asp Asn Ile His Asp Cys Ser Lys Leu Lys Ser Pro Leu Gly
   185 190 195 200
   gtc aaa tgg cat acg gca gtc acc tat gtg aac agc tgc ttg ttt gtg
   976
   Val Lys Trp His Thr Ala Val Thr Tyr Val Asn Ser Cys Leu Phe Val
   205 210 215
   gcc gtg ctg gtg att ctg atc gga tgt tac ata gcc ata tcc agg tac
   1024
   Ala Val Leu Val Ile Leu Ile Gly Cys Tyr Ile Ala Ile Ser Arg Tyr
   220                 225                 230
   atc cac aaa tcc agc agg caa ttc ata agt cag tca agc cga aag cga
   1072
   Ile His Lys Ser Ser Arg Gln Phe Ile Ser Gln Ser Ser Arg Lys Arg
           235                 240                 245
   aaa cat aac cag agc atc agg gtt gtt gtg gct gtg ttt ttt acc tgc
   1120
   Lys His Asn Gln Ser Ile Arg Val Val Val Ala Val Phe Phe Thr Cys
       250                 255                 260
   ttt cta cca tat cac ttg tgc aga att cct ttt act ttt agt cac tta
   1168
   Phe Leu Pro Tyr His Leu Cys Arg Ile Pro Phe Thr Phe Ser His Leu
   265                 270                 275                 280
   gac agg ctt tta gat gaa tct gca caa aaa atc cta tat tac tgc aaa
   1216
   Asp Arg Leu Leu Asp Glu Ser Ala Gln Lys Ile Leu Tyr Tyr Cys Lys
                   285                 290                 295
   gaa att aca ctt ttc ttg tct gcg tgt aat gtt tgc ctg gat cca ata
   1264
   Glu Ile Thr Leu Phe Leu Ser Ala Cys Asn Val Cys Leu Asp Pro Ile
               300                 305                 310
   att tac ttt ttc atg tgt agg tca ttt tca aga agg ctg ttc aaa aaa
   1312
   Ile Tyr Phe Phe Met Cys Arg Ser Phe Ser Arg Arg Leu Phe Lys Lys
           315                 320                 325
   tca aat atc aga acc agg agt gaa agc atc aga tca ctg caa agt gtg
   1360
   Ser Asn Ile Arg Thr Arg Ser Glu Ser Ile Arg Ser Leu Gln Ser Val
       330                 335                 340
   aga aga tcg gaa gtt cgc ata tat tat gat tac act gat gtg tag
   1405
   Arg Arg Ser Glu Val Arg Ile Tyr Tyr Asp Tyr Thr Asp Val  *
   345                 350                 355
   gccttttatt gtttgttgga atcgatatgt acaaagtgta aataaatgtt tcttttcatt
   1465
   aataaaamaa aaaaaaaaaa aaag
   1489

<210> 3
<211> 359
<212> PRT
<213> Mus musculus

<400> 3
Met Ala Val Pro Asn Val Asn Val Ser Thr Phe Ala Gly Asn Glu Leu
 1               5                  10                  15

Tyr Ser Gln Ala Ser His Thr Ala Asn Ser Thr Ser Glu Gly His Gly
            20                  25                  30
Lys Asn Ser Thr Leu His Asn Lys Phe Asp Thr Ile Ile Leu Pro Val
        35                  40                  45
Leu Tyr Leu Val Ile Phe Val Ala Ser Ile Leu Leu Asn Gly Leu Ala
    50                  55                  60
Val Trp Ile Phe Phe His Ile Arg Asn Lys Thr Ser Phe Ile Phe Tyr
65                  70                  75                  80
Leu Lys Asn Ile Val Val Ala Asp Leu Ile Met Thr Leu Thr Phe Pro
                85                  90                  95
Phe Arg Ile Val Arg Asp Ala Gly Phe Gly Pro Trp Tyr Phe Glu Phe
            100                 105                 110
Ile Leu Cys Arg Tyr Thr Ser Val Leu Phe Tyr Ala Asn Met Tyr Thr
        115                 120                 125
Ser Ile Val Phe Leu Gly Leu Ile Ser Val Asp Arg Tyr Leu Lys Val
    130                 135                 140
Val Lys Pro Phe Gly Asp Ser Arg Met Tyr Ser Ile Thr Phe Thr Lys
145                 150                 155                 160
Val Leu Ser Val Cys Val Trp Val Ile Met Ala Ile Leu Ser Leu Pro
                165                 170                 175
Asn Ile Ile Leu Thr Asn Gly Gln Pro Thr Lys Glu Asn Ile His Asp
            180                 185                 190
Cys Met Lys Leu Lys Ser Pro Leu Gly Ala Lys Trp His Met Ala Val
        195                 200                 205
Thr Tyr Val Asp Ser Cys Leu Phe Val Ala Val Leu Val Ile Leu Ile
    210                 215                 220
Gly Cys Tyr Ile Ala Ile Ser Arg Tyr Ile His Lys Ser Ser Arg Gln
225                 230                 235                 240
Phe Ile Ser Gln Ser Ser Arg Lys Arg Lys His Asn Gln Ser Ile Arg
                245                 250                 255
Val Val Val Ala Val Phe Phe Thr Cys Phe Leu Pro Tyr His Leu Cys
            260                 265                 270
Arg Ile Pro Phe Thr Phe Ser Asn Leu Asp Arg Leu Leu Asp Glu Ser
        275                 280                 285
Ala His Lys Ile Leu Tyr Tyr Cys Lys Glu Met Thr Leu Phe Leu Ser
    290                 295                 300
Ala Cys Asn Val Cys Leu Asp Pro Ile Ile Tyr Phe Phe Met Cys Lys
305                 310                 315                 320
Ser Phe Ser Arg Arg Leu Phe Lys Lys Ser Asn Ile Arg Thr Arg Ser
                325                 330                 335
Glu Ser Ile Arg Ser Leu Gln Ser Val Arg Arg Ser Glu Val Arg Ile
            340                 345                 350
Tyr Tyr Asp Tyr Thr Asp Val
        355

<210> 4
<211> 338
<212> PRT
<213> Mus musculus

<400> 4
Met Asn Asn Ser Thr Thr Thr Asp Pro Pro Asn Gln Pro Cys Ser Trp
 1               5                  10                  15
Asn Thr Leu Ile Thr Lys Gln Ile Ile Pro Val Leu Tyr Gly Met Val

                20                  25                  30
Phe Ile Thr Gly Leu Leu Leu Asn Gly Ile Ser Gly Trp Ile Phe Phe
            35                  40                  45
Tyr Val Pro Ser Ser Lys Ser Phe Ile Ile Tyr Leu Lys Asn Ile Val
    50                  55                  60
Val Ala Asp Phe Leu Met Gly Leu Thr Phe Pro Phe Lys Val Leu Gly
65                  70                  75                  80
Asp Ser Gly Leu Gly Pro Trp Gln Val Asn Val Phe Val Cys Arg Val
                85                  90                  95
Ser Ala Val Ile Phe Tyr Val Asn Met Tyr Val Ser Ile Val Phe Phe
            100                 105                 110
Gly Leu Ile Ser Phe Asp Arg Tyr Tyr Lys Ile Val Lys Pro Leu Leu
        115                 120                 125
Thr Ser Ile Val Gln Ser Val Asn Tyr Ser Lys Leu Leu Ser Val Leu
    130                 135                 140
Val Trp Met Leu Met Leu Leu Leu Ala Val Pro Asn Ile Ile Leu Thr
145                 150                 155                 160
Asn Gln Gly Val Lys Glu Val Thr Lys Ile Gln Cys Met Glu Leu Lys
                165                 170                 175
Asn Glu Leu Gly Arg Lys Trp His Lys Ala Ser Asn Tyr Ile Phe Val
            180                 185                 190
Ser Ile Phe Trp Val Val Phe Leu Leu Leu Ile Val Phe Tyr Thr Ala
        195                 200                 205
Ile Thr Arg Lys Ile Phe Lys Ser His Leu Lys Ser Arg Lys Asn Ser
    210                 215                 220
Thr Ser Val Lys Arg Lys Ser Ser Arg Asn Ile Phe Ser Ile Val Leu
225                 230                 235                 240
Val Phe Val Val Cys Phe Val Pro Tyr His Ile Ala Arg Ile Pro Tyr
                245                 250                 255
Thr Lys Ser Gln Thr Glu Gly His Tyr Ser Cys Arg Thr Lys Glu Thr
            260                 265                 270
Leu Leu Tyr Ala Lys Glu Phe Thr Leu Leu Leu Ser Ala Ala Asn Val
        275                 280                 285
Cys Leu Asp Pro Ile Ile Tyr Phe Phe Leu Cys Gln Pro Phe Arg Glu
    290                 295                 300
Val Leu Asn Lys Lys Leu His Met Ser Leu Lys Val Gln Asn Asp Leu
305                 310                 315                 320
Glu Val Ser Lys Thr Lys Arg Glu Asn Ala Ile His Glu Ser Thr Asp
                325                 330                 335
Thr Leu

<210> 5
<211> 305
<212> PRT
<213> Rattus norvegicus

<400> 5
Met Asp Asn Thr Thr Thr Thr Glu Pro Pro Lys Gln Pro Cys Thr Arg
 1               5                  10                  15
Asn Thr Leu Ile Thr Gln Gln Ile Ile Pro Met Leu Tyr Cys Val Val
                20                  25                  30
Phe Ile Thr Gly Val Leu Leu Asn Gly Ile Ser Gly Trp Ile Phe Phe
            35                  40                  45

Tyr Val Pro Ser Ser Lys Ser Phe Ile Ile Tyr Leu Lys Asn Ile Val
    50                  55                  60
Val Ala Asp Phe Leu Met Gly Leu Thr Phe Pro Phe Lys Val Leu Ser
65                  70                  75                  80
Asp Ser Gly Leu Gly Pro Trp Gln Leu Asn Val Phe Val Phe Arg Val
                85                  90                  95
Ser Ala Val Ile Phe Tyr Val Asn Met Tyr Val Ser Ile Ala Phe Phe
            100                 105                 110
Gly Leu Ile Ser Phe Asp Arg Tyr Tyr Lys Ile Val Lys Pro Leu Leu
        115                 120                 125
Val Ser Ile Val Gln Ser Val Asn Tyr Ser Lys Val Leu Ser Val Leu
    130                 135                 140
Val Trp Val Leu Met Leu Leu Leu Ala Val Pro Asn Ile Ile Leu Thr
145                 150                 155                 160
Asn Gln Ser Val Lys Asp Val Thr Asn Ile Gln Cys Met Glu Leu Lys
                165                 170                 175
Asn Glu Leu Gly Arg Lys Trp His Lys Ala Ser Asn Tyr Val Phe Val
            180                 185                 190
Ser Ile Phe Trp Ile Val Phe Leu Leu Leu Thr Val Phe Tyr Met Ala
        195                 200                 205
Ile Thr Arg Lys Ile Phe Lys Ser His Leu Lys Ser Arg Lys Asn Ser
    210                 215                 220
Ile Ser Val Lys Arg Lys Ser Ser Arg Asn Ile Phe Ser Ile Val Leu
225                 230                 235                 240
Ala Phe Val Ala Cys Phe Ala Pro Tyr His Val Ala Arg Ile Pro Tyr
                245                 250                 255
Thr Lys Ser Gln Thr Glu Gly His Tyr Ser Cys Gln Ala Lys Glu Thr
            260                 265                 270
Leu Leu Tyr Thr Lys Glu Phe Thr Leu Leu Leu Ser Ala Ala Asn Val
        275                 280                 285
Cys Leu Asp Pro Ile Ser Ile Ser Ser Tyr Ala Ser Arg Leu Glu Lys
    290                 295                 300
Ser
305

<210> 6
<211> 338
<212> PRT
<213> Homo sapiens

<400> 6
Met Ile Asn Ser Thr Ser Thr Gln Pro Pro Asp Glu Ser Cys Ser Gln
 1               5                  10                  15
Asn Leu Leu Ile Thr Gln Gln Ile Ile Pro Val Leu Tyr Cys Met Val
            20                  25                  30
Phe Ile Ala Gly Ile Leu Leu Asn Gly Val Ser Gly Trp Ile Phe Phe
        35                  40                  45
Tyr Val Pro Ser Ser Lys Ser Phe Ile Ile Tyr Leu Lys Asn Ile Val
    50                  55                  60
Ile Ala Asp Phe Val Met Ser Leu Thr Phe Pro Phe Lys Ile Leu Gly
65                  70                  75                  80
Asp Ser Gly Leu Gly Pro Trp Gln Leu Asn Val Phe Val Cys Arg Val
                85                  90                  95
Ser Ala Val Leu Phe Tyr Val Asn Met Tyr Val Ser Ile Val Phe Phe

                100                 105                 110
Gly Leu Ile Ser Phe Asp Arg Tyr Tyr Lys Ile Val Lys Pro Leu Trp
        115                 120                 125
Thr Ser Phe Ile Gln Ser Val Ser Tyr Ser Lys Leu Leu Ser Val Ile
    130                 135                 140
Val Trp Met Leu Met Leu Leu Leu Ala Val Pro Asn Ile Ile Leu Thr
145                 150                 155                 160
Asn Gln Ser Val Arg Glu Val Thr Gln Ile Lys Cys Ile Glu Leu Lys
                165                 170                 175
Ser Glu Leu Gly Arg Lys Trp His Lys Ala Ser Asn Tyr Ile Phe Val
            180                 185                 190
Ala Ile Phe Trp Ile Val Phe Leu Leu Leu Ile Val Phe Tyr Thr Ala
        195                 200                 205
Ile Thr Lys Lys Ile Phe Lys Ser His Leu Lys Ser Ser Arg Asn Ser
    210                 215                 220
Thr Ser Val Lys Lys Lys Ser Ser Arg Asn Ile Phe Ser Ile Val Phe
225                 230                 235                 240
Val Phe Phe Val Cys Phe Val Pro Tyr His Ile Ala Arg Ile Pro Tyr
                245                 250                 255
Thr Lys Ser Gln Thr Glu Ala His Tyr Ser Cys Gln Ser Lys Glu Ile
            260                 265                 270
Leu Arg Tyr Met Lys Glu Phe Thr Leu Leu Leu Ser Ala Ala Asn Val
        275                 280                 285
Cys Leu Asp Pro Ile Ile Tyr Phe Phe Leu Cys Gln Pro Phe Arg Glu
    290                 295                 300
Ile Leu Cys Lys Lys Leu His Ile Pro Leu Lys Ala Gln Asn Asp Leu
305                 310                 315                 320
Asp Ile Ser Arg Ile Lys Arg Gly Asn Thr Thr Leu Glu Ser Thr Asp
                325                 330                 335
Thr Leu

<210> 7
<211> 347
<212> PRT
<213> Mus musculus

<400> 7
Met Asp Val Pro Gly Val Asn Thr Thr Ser Ala Asn Thr Thr Phe Ser
 1               5                  10                  15
Pro Gly Thr Ser Thr Leu Cys Val Arg Asp Tyr Lys Ile Thr Gln Val
                20                  25                  30
Leu Phe Pro Leu Leu Tyr Thr Val Leu Phe Phe Ala Gly Leu Ile Thr
        35                  40                  45
Asn Ser Leu Ala Met Arg Ile Phe Phe Gln Ile Arg Ser Lys Ser Asn
    50                  55                  60
Phe Ile Ile Phe Leu Lys Asn Thr Val Ile Ser Asp Leu Leu Met Ile
65                  70                  75                  80
Leu Thr Phe Pro Phe Lys Ile Leu Ser Asp Ala Lys Leu Gly Ala Gly
                85                  90                  95
Pro Leu Arg Thr Leu Val Cys Gln Val Thr Ser Val Thr Phe Tyr Phe
                100                 105                 110
Thr Met Tyr Ile Ser Ile Ser Phe Leu Gly Leu Ile Thr Ile Asp Arg
        115                 120                 125

Tyr Leu Lys Thr Thr Arg Pro Phe Lys Thr Ser Ser Pro Ser Asn Leu
    130                 135                 140
Leu Gly Ala Lys Ile Leu Ser Val Val Ile Trp Ala Phe Met Phe Leu
145                 150                 155                 160
Ile Ser Leu Pro Asn Met Ile Leu Thr Asn Arg Arg Pro Lys Asp Lys
                165                 170                 175
Asp Val Thr Lys Cys Ser Phe Leu Lys Ser Glu Phe Gly Leu Val Trp
            180                 185                 190
His Glu Ile Val Asn Tyr Ile Cys Gln Val Ile Phe Trp Ile Asn Phe
        195                 200                 205
Leu Ile Val Ile Val Cys Tyr Ser Leu Ile Thr Lys Glu Leu Tyr Arg
    210                 215                 220
Ser Tyr Val Arg Thr Arg Gly Ser Ala Lys Val Pro Lys Lys Lys Val
225                 230                 235                 240
Asn Val Lys Val Phe Ile Ile Ile Ala Val Phe Phe Ile Cys Phe Val
                245                 250                 255
Pro Phe His Phe Ala Arg Ile Pro Tyr Thr Leu Ser Gln Thr Arg Ala
            260                 265                 270
Val Phe Asp Cys Ser Ala Glu Asn Thr Leu Phe Tyr Val Lys Glu Ser
        275                 280                 285
Thr Leu Trp Leu Thr Ser Leu Asn Ala Cys Leu Asp Pro Phe Ile Tyr
    290                 295                 300
Phe Phe Leu Cys Lys Ser Phe Arg Asn Ser Leu Thr Ser Met Leu Arg
305                 310                 315                 320
Cys Ser Asn Ser Thr Ser Thr Ser Gly Thr Asn Lys Lys Lys Gly Gln
                325                 330                 335
Glu Gly Gly Glu Pro Ser Glu Glu Thr Pro Met
            340                 345

<210> 8
<211> 343
<212> PRT
<213> Rattus norvegicus

<400> 8
Met Glu Val Pro Gly Ala Asn Ala Thr Ser Ala Asn Thr Thr Ser Ile
 1               5                  10                  15
Pro Gly Thr Ser Thr Leu Cys Ser Arg Asp Tyr Lys Ile Thr Gln Val
            20                  25                  30
Leu Phe Pro Leu Leu Tyr Thr Val Leu Phe Phe Ala Gly Leu Ile Thr
        35                  40                  45
Asn Ser Leu Ala Met Arg Ile Phe Phe Gln Ile Arg Ser Lys Ser Asn
    50                  55                  60
Phe Ile Ile Phe Leu Lys Asn Thr Val Ile Ser Asp Leu Leu Met Ile
65                  70                  75                  80
Leu Thr Phe Pro Phe Lys Ile Leu Ser Asp Ala Lys Leu Gly Ala Gly
                85                  90                  95
His Leu Arg Thr Leu Val Cys Gln Val Thr Ser Val Thr Phe Tyr Phe
            100                 105                 110
Thr Met Tyr Ile Ser Ile Ser Phe Leu Gly Leu Ile Thr Ile Asp Arg
        115                 120                 125
Tyr Leu Lys Thr Thr Arg Pro Phe Lys Thr Ser Ser Pro Ser Asn Leu
    130                 135                 140
Leu Gly Ala Lys Ile Leu Ser Val Ala Ile Trp Ala Phe Met Phe Leu

145                 150                 155                 160
Leu Ser Leu Pro Asn Met Ile Leu Thr Asn Arg Arg Pro Lys Asp Lys
                165                 170                 175
Asp Ile Thr Lys Cys Ser Phe Leu Lys Ser Glu Phe Gly Leu Val Trp
            180                 185                 190
His Glu Ile Val Asn Tyr Ile Cys Gln Val Ile Phe Trp Ile Asn Phe
        195                 200                 205
Leu Ile Val Ile Val Cys Tyr Ser Leu Ile Thr Lys Glu Leu Tyr Arg
    210                 215                 220
Ser Tyr Val Arg Thr Arg Gly Ser Ala Lys Ala Pro Lys Lys Arg Val
225                 230                 235                 240
Asn Ile Lys Val Phe Ile Ile Ile Ala Val Phe Phe Ile Cys Phe Val
                245                 250                 255
Pro Phe His Phe Ala Arg Ile Pro Tyr Thr Leu Ser Gln Thr Arg Ala
            260                 265                 270
Val Phe Asp Cys Asn Ala Glu Asn Thr Leu Phe Tyr Val Lys Glu Ser
        275                 280                 285
Thr Leu Trp Leu Thr Ser Leu Asn Ala Cys Leu Asp Pro Phe Ile Tyr
    290                 295                 300
Phe Phe Leu Cys Lys Ser Phe Arg Asn Ser Leu Met Ser Met Leu Arg
305                 310                 315                 320
Cys Ser Thr Ser Gly Ala Asn Lys Lys Lys Gly Gln Glu Gly Gly Asp
                325                 330                 335
Pro Ser Glu Glu Thr Pro Met
            340


<210> 9
<211> 342
<212> PRT
<213> Homo sapiens

<400> 9

<210> 10
<211> 337
<212> PRT
<213> Mus musculus

<400> 10
Met Leu Gly Thr Ile Asn Thr Thr Gly Met Gln Gly Phe Asn Lys Ser
1               5                   10                  15
Glu Arg Cys Pro Arg Asp Thr Arg Met Thr Gln Leu Leu Phe Pro Val
            20                  25                  30
Leu Tyr Thr Val Val Phe Leu Ala Gly Ile Leu Leu Asn Thr Val Ala
        35                  40                  45
Leu Trp Val Phe Val His Ile Pro Ser Asn Ser Thr Phe Ile Val Tyr
    50                  55                  60
Leu Lys Asn Thr Leu Val Ala Asp Leu Ile Met Ala Leu Met Leu Pro
65                  70                  75                  80
Phe Lys Ile Leu Ser Asp Ser His Leu Ala Pro Trp Gln Leu Arg Gly
                85                  90                  95
Phe Val Cys Thr Leu Ser Ser Val Val Phe Tyr Glu Thr Met Tyr Val
            100                 105                 110
Gly Ile Met Met Leu Gly Leu Ile Ala Phe Asp Arg Phe Leu Lys Ile
        115                 120                 125
Ile Met Pro Phe Arg Lys Thr Phe Val Lys Lys Thr Ala Phe Ala Lys
    130                 135                 140
Thr Val Ser Ile Ser Val Trp Ser Leu Met Phe Phe Ile Ser Leu Pro
145                 150                 155                 160
Asn Met Ile Leu Asn Lys Glu Ala Thr Pro Ser Ser Val Lys Lys Cys
                165                 170                 175
Ala Ser Leu Lys Ser Pro Leu Gly Leu Trp Trp His Gln Val Val Ser
            180                 185                 190
His Thr Cys Gln Leu Ile Phe Trp Ala Val Phe Ile Leu Met Leu Leu

            195                 200                 205
Phe Tyr Ala Val Ile Thr Lys Lys Val Tyr Asn Ser Tyr Arg Lys Phe
    210                 215                 220
Arg Ser Lys Asp Ser Arg His Lys Arg Leu Glu Val Lys Val Phe Ile
225                 230                 235                 240
Val Met Ala Val Phe Phe Val Cys Phe Ala Pro Leu His Phe Val Arg
                245                 250                 255
Ile Pro Tyr Thr Tyr Ser Gln Thr Thr Asn Lys Thr Asp Cys Arg Leu
            260                 265                 270
Glu Asn Gln Leu Phe Ile Ala Lys Glu Ala Thr Leu Phe Leu Ala Thr
        275                 280                 285
Thr Asn Ile Cys Met Asp Pro Leu Ile Tyr Ile Ile Leu Cys Lys Lys
    290                 295                 300
Phe Thr Gln Lys Val Pro Cys Val Arg Trp Gly Lys Ala Arg Thr Ala
305                 310                 315                 320
Gly Ser Ser Glu Asp His His Ser Ser Gln Thr Asp Asn Ile Thr Leu
                325                 330                 335
Ala

<210> 11
<211> 333
<212> PRT
<213> Homo sapiens

<400> 11
Met Asn Thr Thr Val Met Gln Gly Phe Asn Arg Ser Glu Arg Cys Pro
 1               5                  10                  15
Arg Asp Thr Arg Ile Val Gln Leu Val Phe Pro Ala Leu Tyr Thr Val
            20                  25                  30
Val Phe Leu Thr Gly Ile Leu Leu Asn Thr Leu Ala Leu Trp Val Phe
        35                  40                  45
Val His Ile Pro Ser Ser Ser Thr Phe Ile Ile Tyr Leu Lys Asn Thr
    50                  55                  60
Leu Val Ala Asp Leu Ile Met Thr Leu Met Leu Pro Phe Lys Ile Leu
65                  70                  75                  80
Ser Asp Ser His Leu Ala Pro Trp Gln Leu Arg Ala Phe Val Cys Arg
                85                  90                  95
Phe Ser Ser Val Ile Phe Tyr Glu Thr Met Tyr Val Gly Ile Val Leu
            100                 105                 110
Leu Gly Leu Ile Ala Phe Asp Arg Phe Leu Lys Ile Ile Arg Pro Leu
        115                 120                 125
Arg Asn Ile Phe Leu Lys Lys Pro Val Phe Ala Lys Thr Val Ser Ile
    130                 135                 140
Phe Ile Trp Phe Phe Leu Phe Phe Ile Ser Leu Pro Asn Thr Ile Leu
145                 150                 155                 160
Ser Asn Lys Glu Ala Thr Pro Ser Ser Val Lys Lys Cys Ala Ser Leu
                165                 170                 175
Lys Gly Pro Leu Gly Leu Lys Trp His Gln Met Val Asn Asn Ile Cys
            180                 185                 190
Gln Phe Ile Phe Trp Thr Val Phe Ile Leu Met Leu Val Phe Tyr Val
        195                 200                 205
Val Ile Ala Lys Lys Val Tyr Asp Ser Tyr Arg Lys Ser Lys Ser Lys
    210                 215                 220

Asp Arg Lys Asn Asn Lys Lys Leu Glu Gly Lys Val Phe Val Val Val
225                 230                 235                 240
Ala Val Phe Phe Val Cys Phe Ala Pro Phe His Phe Ala Arg Val Pro
                245                 250                 255
Tyr Thr His Ser Gln Thr Asn Asn Lys Thr Asp Cys Arg Leu Gln Asn
            260                 265                 270
Gln Leu Phe Ile Ala Lys Glu Thr Thr Leu Phe Leu Ala Ala Thr Asn
        275                 280                 285
Ile Cys Met Asp Pro Leu Ile Tyr Ile Phe Leu Cys Lys Lys Phe Thr
    290                 295                 300
Glu Lys Leu Pro Cys Met Gln Gly Arg Lys Thr Thr Ala Ser Ser Gln
305                 310                 315                 320
Glu Asn His Ser Ser Gln Thr Asp Asn Ile Thr Leu Gly
                325                 330

<210> 12
<211> 20
<212> DNA
<213> Artificial Sequence

<220>
<223> 2871 forward primer

<400> 12
atcgtgttcc ttgggctgat
20

<210> 13
<211> 19
<212> DNA
<213> Artificial Sequence

<220>
<223> 2871 reverse primer

<400> 13
tccgagagtc cccaaatgg
19

<210> 14
<211> 29
<212> DNA
<213> Artificial Sequence

<220>
<223> 2871 probe

<400> 14
agcattgatc gctatctgaa ggtggtcaa
29